# EUROPEAN PATENT SPECIFICATION

(11) **EP 3 563 874 B1**
(45) Date of publication and mention of the grant of the patent: **22.11.2023**
(21) Application number: 19172114.1
(22) Date of filing: 01.05.2019
(51) Int. Cl.: A61K 49/18, A61K 49/00, A61K 51/12, A61K 103/00, B82Y 5/00

(54) **A MULTIMODAL PET/MRI CONTRAST AGENT AND A PROCESS FOR THE SYNTHESIS THEREOF**
MULTIMODALES PET/MRT-KONTRASTMITTEL UND VERFAHREN ZUR SYNTHESE DAVON
AGENT DE CONTRASTE MULTIMODAL POUR TEP/IRM ET SON PROCÉDÉ DE SYNTHÈSE

(30) Priority: 02.05.2018 AU 2018901483
(43) Date of publication of application: 06.11.2019
(73) Proprietor: Royal Melbourne Institute Of Technology, Melbourne, Victoria 3000 (AU)
(72) Inventor: ANDERSON, Amanda, Eltham, Victoria 3095 (AU); BANSAL, Vipul, Heidelberg West, Victoria 3081 (AU); CAMPBELL, Jos Laurie, Mountain View, California 94043 (US); RAMANATHAN, Rajesh, Malvern East, Victoria 3145 (AU); SHUKLA, Ravi, Rosanna, Victoria 3084 (AU); ARORA, Jyoti, Ringwood, Victoria 3134 (AU)
(74) Representative: Marks & Clerk LLP

(56) References cited:
- WO-A1-2016/191247
- US-A1- 2004 192 998
- AFSANEH LAHOOTI ET AL: "Dual nano-sized contrast agents in PET/MRI: a systematic review : Dual Contrast Agents In PET/MRI", CONTRAST MEDIA & MOLECULAR IMAGING, vol. 11, no. 6, 1 November 2016 (2016-11-01), pages 428-447, XP055622001, GB ISSN: 1555-4309, DOI: 10.1002/cmmi.1719
- CHANG-TONG YANG ET AL: "PET-MR and SPECT-MR multimodality probes: Development and challenges", THERANOSTICS, vol. 8, no. 22, 29 November 2018 (2018-11-29), pages 6210-6232, XP055622109, AU ISSN: 1838-7640, DOI: 10.7150/thno.26610
- LYDIA SANDIFORD ET AL: "Bisphosphonate-Anchored PEGylation and Radiolabeling of Superparamagnetic Iron Oxide: Long-Circulating Nanoparticles for in Vivo Multimodal (T1 MRI-SPECT) Imaging", ACS NANO, vol. 7, no. 1, 29 November 2012 (2012-11-29), pages 500-512, XP055316494, US ISSN: 1936-0851, DOI: 10.1021/nn3046055
- JIN-SIL CHOI ET AL: "A Hybrid Nanoparticle Probe for Dual-Modality Positron Emission Tomography and Magnetic Resonance Imaging", ANGEWANDTE CHEMIE INTERNATIONAL EDITION, vol. 47, no. 33, 4 August 2008 (2008-08-04), pages 6259-6262, XP055144623, ISSN: 1433-7851, DOI: 10.1002/anie.200801369
- RIPEN MISRI ET AL: "Development and evaluation of a dual-modality (MRI/SPECT) molecular imaging bioprobe", NANOMEDICINE: NANOTECHNOLOGY, BIOLOGY AND MEDICINE, vol. 8, no. 6, 1 August 2012 (2012-08-01), pages 1007-1016, XP055622285, NL ISSN: 1549-9634, DOI: 10.1016/j.nano.2011.10.013
- RAMESH SHARMA ET AL: "Carbon-11 radiolabeling of iron-oxide nanoparticles for dual-modality PET/MR imaging", NANOSCALE, vol. 5, no. 16, 1 January 2013 (2013-01-01), page 7476, XP055622284, United Kingdom ISSN: 2040-3364, DOI: 10.1039/c3nr02519e
- Renata Madru ET AL: "68Ga-labeled superparamagnetic iron oxide nanoparticles (SPIONs) for multi modality PET/MR/Cherenkov luminescence imaging of sentinel lymph nodes", Am J Nucl Med Mol Imaging, 1 January 2014 (2014-01-01), XP055622281, Retrieved from the Internet: URL:https://www.ncbi.nlm.nih.gov/pmc/artic les/PMC3867730/pdf/ajnmmi0004-0060.pdf [retrieved on 2019-09-13]
- TSIAPA IRENE ET AL: "99mTc-labeled aminosilane-coated iron oxide nanoparticles for molecular imaging of[alpha][nu][beta]3-mediated tumor expression and feasibility for hypertherm", JOURNAL OF COLLOID AND INTERFACE SCIENCE, ACADEMIC PRESS,INC, US, vol. 433, 1 August 2014 (2014-08-01), pages 163-175, XP029054894, ISSN: 0021-9797, DOI: 10.1016/J.JCIS.2014.07.032
- NAHRENDORF M ET AL: "Detection of macrophages in aortic aneurysms by nanoparticle positron emission tomography-computed tomography", ARTERIOSCLEROSIS, THROMBOSIS, AND VASCULAR BIOLOGY, LIPPINCOTT, WILLIAMS & WILKINS , vol. 31, no. 4 1 January 2011 (2011-01-01), pages 750-757, XP008141229, ISSN: 1079-5642, DOI: 10.1161/ATVBAHA.110.221499 Retrieved from the Internet: URL:http://atvb.ahajournals.org/
- LARS STELTER ET AL: "Modification of Aminosilanized Superparamagnetic Nanoparticles: Feasibility of Multimodal Detection Using 3T MRI, Small Animal PET, and Fluorescence Imaging", MOLECULAR IMAGING AND BIOLOGY, SPRINGER-VERLAG, NE, vol. 12, no. 1, 7 July 2009 (2009-07-07), pages 25-34, XP019775811, ISSN: 1860-2002
- CUI XIANJIN ET AL: "Aluminium hydroxide stabilised MnFe2O4and Fe3O4nanoparticles as dual-modality contrasts agent for MRI and PET imaging", BIOMATERIALS, ELSEVIER SCIENCE PUBLISHERS BV., BARKING, GB, vol. 35, no. 22, 24 April 2014 (2014-04-24), pages 5840-5846, XP028660801, ISSN: 0142-9612, DOI: 10.1016/J.BIOMATERIALS.2014.04.004
- JEONG CHAN PARK ET AL: "Facile Preparation of a Hybrid Nanoprobe for Triple-Modality Optical/PET/MR Imaging", SMALL, vol. 6, no. 24, 20 December 2010 (2010-12-20), pages 2863-2868, XP055622283, DE ISSN: 1613-6810, DOI: 10.1002/smll.201001418
- DELGADO GARCIA ET AL: "Development of a new multimodal diagnostic nanosystem based on inorganic nanoparticles as a potential radiopharmaceutical in SPECT-MRI or PET-MRI imaging in: Annual Congress of the EANM Abstracts (pages S1-S734)", EUROPEAN JOURNAL OF NUCLEAR MEDICINE AND MOLECULAR IMAGING, vol. 43, no. Suppl 1, EP532, 21 September 2016 (2016-09-21), pages S431-S432, XP036069311, ISSN: 1619-7070, DOI: 10.1007/S00259-016-3484-4 [retrieved on 2016-09-21]
- TRAVIS M. SHAFFER ET AL: "Silica Nanoparticles as Substrates for Chelator-free Labeling of Oxophilic Radioisotopes", NANO LETTERS, vol. 15, no. 2, 15 January 2015 (2015-01-15), pages 864-868, XP055523167, US ISSN: 1530-6984, DOI: 10.1021/nl503522y
- SLAVKO KRALJ ET AL: "Producing ultra-thin silica coatings on iron-oxide nanoparticles to improve their surface reactivity", JOURNAL OF MAGNETISM AND MAGNETIC MATERIALS, vol. 322, no. 13, 1 July 2010 (2010-07-01) , pages 1847-1853, XP055621999, AMSTERDAM, NL ISSN: 0304-8853, DOI: 10.1016/j.jmmm.2009.12.038

## Description

### Technical Field

The present invention relates to multimodal PET (positron emission tomography)/MRI (magnetic resonance imaging) contrast agents for image enhancement, and more particularly to a multimodal PET (positron emission tomography)/MRI (magnetic resonance imaging) contrast agent that does not require a chelator or complexing agent to bind the radionuclide responsible for providing the PET signal.

The invention has been developed primarily for use as a multimodal contrast agent in PET (positron emission tomography)/MRI (magnetic resonance imaging) imaging, and will be described hereinafter with reference to this application.

The following discussion of the background to the invention is intended to facilitate an understanding of the invention. However, it should be appreciated that the discussion is not an acknowledgement or admission that any of the material referred to was published, known or part of the common general knowledge in Australia or any other country as at the priority date of any one of the claims of this specification.

### Background of Invention

To date, a number of techniques have been employed as a means of non-invasively providing medical images. There is no one ultimate method of imaging that is capable of providing a detailed image of all fractions of the body (blood, bone, organ, tissue, cells). Therefore, each technique fills a particular niche and cohesively along with other imaging techniques, provides new and complementary information in order to understand the big picture. In recent years there has been a push for non-invasive methods and multimodal methods of imaging that may be capable of providing a more detailed image as a result. Despite this constant evolution of technology, the ability to produce images of internal structures of the body still remains limited.

Magnetic resonance imaging (MRI) is an imaging modality that is used to construct images of the nuclear magnetic resonance (NMR) signal, primarily from the hydrogen atoms in an object. The image contrast is achieved by the differences in the NMR signal intensity in different areas within the object, and the NMR signal intensity largely depends on the nuclear density (proton spins), the relaxation times (T1, T2, and T2*), and the magnetic environment of the tissues.

Contrast agents are a group of contrast media used to improve the visibility of internal body structures in magnetic resonance imaging (MRI).

MRI contrast agents are not directly visible. The modification in contrast is due to their effect of shortening the relaxation time T1 and/or T2 of the hydrogen nuclei located in their vicinity. If the contrast agent reduces time T1 (paramagnetic contrast agents), we observe a T1 hypersignal. On the other hand, if it shortens T2 (superparamagnetic contrast agents), there will be a reduction in the T2 and T2* signal. The effectiveness of the contrast agent depends on its relaxivity (that is, its capacity to modify relaxation times).

The most commonly used compounds for contrast enhancement are gadolinium-based. Such MRI contrast agents shorten the relaxation times of nuclei within body tissues following oral or intravenous administration. In MRI scanners, sections of the body are exposed to a very strong magnetic field causing primarily the hydrogen nuclei ("spins") of water in tissues to be polarized in the direction of the magnetic field. An intense radiofrequency pulse is applied that tips the magnetization generated by the hydrogen nuclei in the direction of the receiver coil where the spin polarization can be detected. Random molecular rotational oscillations matching the resonance frequency of the nuclear spins provide the "relaxation" mechanisms that bring the net magnetization back to its equilibrium position in alignment with the applied magnetic field. The magnitude of the spin polarization detected by the receiver is used to form the MR image but decays with a characteristic time constant known as the T1 relaxation time. Water protons in different tissues have different T1 values, which is one of the main sources of contrast in MR images. A contrast agent usually shortens, but in some instances increases, the value of T1 of nearby water protons thereby altering the contrast in the image.

Positron emission tomography (PET) is another imaging modality in nuclear medicine that is used to observe metabolic processes in the body. The system detects pairs of gamma rays emitted indirectly by a positron-emitting radionuclide (tracer), which is introduced into the body on a biologically active molecule. Three-dimensional images of tracer concentration within the body are then constructed by computer analysis.

Radionuclides used in PET scanning are typically isotopes with short half-lives such as carbon-11 (~20 min), nitrogen-13 (~10 min), oxygen-15 (~2 min), fluorine-18 (∼110 min), gallium-68 (~67 min), zirconium-89 (~78.41 hours), copper-64 (~12.70 hours) or rubidium-82 (~1.27 min). These radionuclides are incorporated either into compounds normally used by the body such as glucose (or glucose analogues), water, or ammonia, or into molecules that bind to receptors or other sites of drug action.

The current use of radionuclides for certain applications is limited by the requirement for the use of chelators or complexing agents to bind these radionuclides to pharmaceuticals, proteins, peptides, targeting agents or directly to biological structures such as cells. Complexing agents and/or chelators operate by providing specific binding sites for the target surface such as a nanoparticle, as well as a specific binding site for the radionuclide. One end of the complexing agent or chelator binds to the core, while the other binds to the radionuclide, thereby providing a mechanism to attach one to the other. As this is a reaction that requires one binding site for each radionuclide, the complexing agent or chelator acts as a significant limiting factor of the reaction. An added complication in the use of chelators is the risk of "poisoning"; this occurs when chelators are exposed to metals unintentionally. This can occur as the result of metals being extracted out of reaction and storage vessels. Once present, these metals can bind to the chelators taking up binding sites and sometimes binding irreversibly. In the case of cell based applications, the current chelator-based radionuclide formulations often suffer from a common lack of efficiency in cell retention and upon *in vivo* administration can lead to significant non-specific uptake.

Magnetic nanomaterials have been commonly used as MRI contrast agents; both T1- and T2-weighted MRI agents have been clinically approved and are commercially available. However, the majority of the T1 contrast agents used at present are gadolinium-based, of which there are several reported side effects, most notably associated with the kidneys including for example, nephrogenic systemic fibrosis (NSF), which are due largely in part to the inherent toxicity of this particular element. For instance, gadolinium(III) is somewhat toxic as a free solubilized aqueous ion, but it is generally regarded as safe when administered as a chelated compound.

Currently, there are no T2-based MRI iron oxide contrast agents being utilised for MRI imaging. Those products that had previously gained marketing approval for use including, Feridex I.V.^{™} (Endorem^{™}; ferumoxides injectable solution), Resovist^{™} (Cliavist^{™}), Sinerem^{™} (Combidex^{™}), Guerbet^{™}, Lumirem^{™} (Gastromark^{™}) and Feraheme^{™} (Ferumoxytol) have since been withdrawn for one reason or another. As the market is dominated by T1 agents for clinical use and common MRI imaging purposes, T2 agents have to date received little market demand. This lack of demand for T2 contrast agents is the primary reason for their withdrawal from the market.

Iron oxide nanoparticles (IONPs) are nanocrystals made from magnetite or hematite. Despite spin surface disorders and the observed spin canting effect, IONPs typically possess substantial saturation magnetization (Ms) values at room temperature, especially for those made from pyrolysis protocols with good crystallinity. Unlike the bulk materials, IONPs that less than 20 nm in size are superparamagnetic - a state where particles show zero magnetism in the absence of an external magnetic field, but can become magnetized when there is one. The underlying mechanism is that at such small scale, the thermal energy is sufficient to overcome the anisotropy energy of each small magnet (nanoparticle), and this leads to random fluctuation of the magnetizations that, macroscopically, result in zero net coercivity and magnetic moment.

The superior magnetic properties of IONPs, along with their inherent biocompatibility and inexpensiveness, have made IONPs a material of choice in many bioapplications, such as contrast probes for magnetic resonance imaging (MRI). The high magnetic moments of IONPs make them effective in reducing T2 relaxation times, leading to signal attenuation on a T2- or T2*-weighted map. When the particles are engineered with targeting specificity, such signal alterations can be harnessed to report abnormal biological activity.

Superparamagnetic iron oxide nanoparticles (SPIONs) comprise a class of novel MRI contrast agents that are composed of a ferric iron (Fe³⁺) and ferrous iron (Fe²⁺) core coated with a layer of polysaccharide such as dextran, or another chemical species. The iron nanoparticles at the core have a very large magnetic moment, which leads to local magnetic field inhomogeneity. Consequently, the intensity of the NMR signal produced in respect of these SPIONs is significantly decreased, appearing dark on T2- and T2*-weighted images. On the basis of size (namely, diameter), SPIONs are commonly classified as oral SPIO (300 nm - 3.5 µm), polydispersed SPIO (PSPIO, 50 nm -150 nm), or ultrasmall SPIO (USPIO, <50 nm). In addition, USPIO nanoparticles with an iron oxide core that is monocrystalline in nature are referred to as monocrystalline iron oxide nanoparticles (MION), and MION with a chemically cross-linked and aminated polysaccharide shell are called cross-linked iron oxide nanoparticles (CLIO).

Over the past few decades, significant technological advances have taken place in an attempt to improve radiologic imaging techniques. One example is the development of the bimodal PET/MRI scanner, which is now finding its way into clinical settings. As the name suggests, the bimodal PET/MRI scanner combines both imaging techniques into a single device. The benefit of this bimodal imaging technique is in its ability to maintain the benefits of each imaging tool individually, thereby producing images of a complementary nature. The bimodal PET/MRI scanner retains excellent sensitivity, high temporal resolution and biological functional imaging through the PET functionalities, whilst high spatial and temporal resolution, details in soft tissue contrast and anatomical information is provided through the MRI imaging modality.

However, despite the fact that a bimodal PET/MRI scanner is commercially available, there are no bimodal or indeed multimodal PET/MRI contrast agents currently available that have received approval from the FDA for use in bimodal PET/MRI imaging in a clinical setting. The access to efficient contrast agents for multimodal PET/MRI imaging would allow appropriate enhancement of the images and potentially improve the utilisation of this technique in the clinic, with the potential to enhance diagnostic accuracy.

The use and development of a number of bimodal or multimodal PET/MRI imaging agents is currently being explored. However, to the best of our knowledge, all of the systems employed to date are still heavily reliant on the use of complexing agents to combine the radionuclides with the nanoparticle core.

Importantly, in regards to current clinical practice, before administration in the patients, radionuclides that have a limited half-life (which means that its efficiency will reduce to half within a certain time, and further exponential decay of activity as time passes), are directly delivered from the source (e.g. a reactor or cyclotron) to the clinic. Technologists or radiochemists then have a very limited time and infrastructure to radiolabel carriers (either particles or chelators) with commercial radionuclides, before administration.

In essence, the majority of the radiometals with physical properties suitable for imaging and/or therapy applications, for example, ⁶⁴Cu, ⁸⁹Zr, ^{99m}Tc, ¹¹¹In, ¹⁷⁷Lu, ⁹⁰Y, require the coordination of certain chelators to form stable complexes. Due to the uniqueness of each radionuclide, knowing the particular coordination chemistry and selecting the best chelator with sufficient in vivo stability are a vital, however, highly challenging task. Therefore, the development of a stable radiopharmaceutical that contains both diagnostic and therapeutic radioisotopes, labeled via a simple but effective chelator-free strategy, is highly desirable.

The present invention seeks to provide a multimodal PET (positron emission tomography)/MRI (magnetic resonance imaging) contrast agent and a process for the synthesis thereof, which will overcome or substantially ameliorate at least some of the deficiencies of the prior art, or to at least provide an alternative.

Lahooti et al. in "Dual nano-sized contrast agents in PET/MRI: a systematic review" (Contrast Media & Molecular Imaging, 2016, vol. 11, no. 6, pp. 428-447) review dual contrast agents in positron emission tomography/magnetic resonance imaging.

Yang et al. in "PET-MR and SPECT-MR multimodality probes: Development and challenges" (Theranostics, 2018, vol. 8, no. 22, pp. 6210-6232) review PET-MR and SPECT-MR multimodal imaging probes.

Sandiford et al. in "Bisphosphonate-Anchored PEGylation and Radiolabeling of Superparamagnetic Iron Oxide: Long-Circulating Nanoparticles for in Vivo Multimodal (T1 MRI-SPECT) Imaging" (ACS Nano, 2012, vol. 7, no. 1, pp. 500-512) disclose a stealth PEG polymer conjugate containing a terminal 1,1-bisphosphonate (BP) group for binding to the surface of ultrasmall-superparamagnetic oxide nanomaterials (USPIOs).

Choi et al. in "A Hybrid Nanoparticle Probe for Dual-Modality Positron Emission Tomography and Magnetic Resonance Imaging" (Angewandte Chemie International Edition, 2008, vol. 47, no. 33, pp. 6259-6262) disclose a probe for combined positron emission tomography and magnetic resonance imaging.

Misri et al. in "Development and evaluation of a dual-modality (MRI/SPECT) molecular imaging bioprobe" (Nanomedicine: Nanotechnology, Biology and Medicine, 2012, vol. 8, no. 6, pp. 1007-1016) disclose the development of dual modality molecular imaging bioprobes, in the form of magnetic nanoparticles (NPs) conjugated to antibodies, for SPECT and MRI of mesothelin-expressing cancers.

Sharma et al. in "Carbon-11 radiolabeling of iron-oxidenanoparticles for dual-modality PET/MR imaging" (Nanoscale, 2013, vol. 5, no. 16, pp. 7476-7483) disclose the development of a nanoparticle labeling procedure via covalent bonding with carbon-11 PET isotope.

Madru et al. in "68Ga-labeled superparamagnetic iron oxide nanoparticles (SPIONs) for multi modality PET/MR/Cherenkov luminescence imaging of sentinel lymph nodes" (Am J Nucl Med Mol Imaging, 2014, vol. 4, no. 1, pp. 60-69) disclose ⁶⁸Ga-SPIONs for use as a single contrast agent for PET/MR imaging of sentinel lymph node.

Irene et al. in "99mTc-labeled aminosilane-coated iron oxide nanoparticles for molecular imaging of αvβ3-mediated tumor expression and feasibility for hyperthermia treatment" (Journal of Colloid and Interface Science, 2014, vol. 433, pp. 163-175) disclose aminosilane coated Fe₃O₄ (10 ± 2 nm) as a tumor imaging agent in nuclear medicine through 3-aminopropyltriethoxysilane (APTES) functionalization.

Nahrendorf et al. in "Detection of Macrophages in Aortic Aneurysms by Nanoparticle Positron Emission Tomography-Computed Tomography" (Atheriosclerosis, Thrombosis and Vascular Biology, 2011, vol. 31, no. 4, pp. 750-757) disclose macrophage-targeted nanoparticles labeled with fluorine-18 (¹⁸F) for positron emission tomography-computed tomography (PET-CT) detection of inflammation in aortic aneurysms.

Stelter et al. in "Modification of Aminosilanized Superparamagnetic Nanoparticles: Feasibility of Multimodal Detection Using 3T MRI, Small Animal PET, and Fluorescence Imaging" (Molecular Imaging and Biology, 2009, vol. 12, no. 1, pp. 25-34) disclose an aminosilane-coated superparamagnetic nanoparticle for cell labelling and subsequent multimodal imaging using magnetic resonance imaging, positron emission tomography and fluorescent imaging in vivo.

Cui et al. in "Aluminium hydroxide stabilised MnFe2O4 and Fe3O4 nanoparticles as dual-modality contrasts agent for MRI and PET imaging" (Biomaterials, 2014, vol. 35, no. 22, pp. 5840-5846) disclose magnetic nanoparticles (NPs) MnFe₂O₄ and Fe₃O₄ that were stabilised by depositing an Al(OH)₃ layer via a hydrolysis process.

Park et al. in "Facile Preparation of a Hybrid Nanoprobe for Triple-Modality Optical/PET/MR Imaging" (Small, 2010, vol. 6, no. 24, pp. 2863-2868) disclose a method of preparing triple-modality, optical-nuclear-magnetic imaging probes using radiolabeled superparamagnetic nanoparticles.

Garcia et al. in "Development of a new multimodal diagnostic nanosystem based on inorganic nanoparticles as a potential radiopharmaceutical in SPECT-MRI or PET-MRI imaging" (European Journal of Nuclear Medicine and Molecular Imaging, 2016, vol. 43, no. Suppl 1, EP 532, pp. S431-S432) disclose a multimodal imaging system (PET-MRI or SPECT-MRI) based on inorganic particles (NPs) and their potential application as a radiopharmaceutical.

Shaffer et al. in "Silica Nanoparticles as Substrates for Chelator-free Labeling of Oxophilic Radioisotopes" (Nano Letters, 2015, vol. 15, no. 2, pp. 864-868) disclose amorphous silica nanoparticles as substrates for chelator-free radiolabelling.

US 2004/192998 A1 discloses radiation delivery devices for use in brachytherapy which employ radioactive palladium-103 as the radiation source material.

WO 2016/191247 A1 discloses a radioactive nanoparticle that comprises a metal nanoparticle core, an outer metal shell disposed over the metal nanoparticle core, and a metallic radioisotope disposed within the metal nanoparticle core or within the outer metal shell.

Kralj et al. in "Producing ultra-thin silica coatings on iron-oxide nanoparticles to improve their surface reactivity" (Journal of Magnetism and Magnetic Materials, 2010, vol. 322, no. 13, pp. 1847-1853) disclose coated superparamagnetic maghemite nanoparticles by hydrolysis and the polycondensation of tetraethyl orthosilicate (TEOS).

### Summary of Invention

According to a first aspect of the present invention there is provided a multimodal PET (positron emission tomography)/MRI (magnetic resonance imaging) contrast agent comprising: a magnetic signal generating core; and a coating portion formed at least partially over a surface of said magnetic signal generating core, wherein the coating portion comprises a plurality of layers, including an inner layer having a functionalized surface, wherein the functionalised surface is silica, and an outer layer in the form of a radionuclide electrolessly plated layer formed on said functionalized surface.

Suitably, the magnetic signal generating core is a ferromagnetic, paramagnetic or superparamagnetic signal generating core.

In one embodiment, the magnetic signal generating core comprises an oxide, a mixed oxide and/or a hydroxide of at least one metal selected from the group consisting of iron (Fe), cobalt (Co), nickel (Ni), neodymium (Nd), gadolinium (Gd) and manganese (Mn).

In one embodiment, the magnetic signal generating core comprises a support material loaded with at least one metal ion selected from the group consisting of iron (Fe), cobalt (Co), nickel (Ni), neodymium (Nd), gadolinium (Gd) and manganese (Mn).

In one embodiment, the magnetic signal generating core comprises an iron-based material selected from the group consisting of iron (Fe), magnetite (Fe₃O₄), maghemite (γ-Fe₂O₃), hematite (α-Fe₂O₃), an iron alloy and a ferrite material.

In one embodiment, the magnetic signal generating core comprises an alloy of at least one metal selected from the group consisting of iron (Fe), cobalt (Co), nickel (Ni), neodymium (Nd), gadolinium (Gd) and manganese (Mn).

In one embodiment, the alloy is an iron alloy selected from the group consisting of gold-iron oxide (Au-Fe₃O₄), iron-cobalt (Fe-Co) and iron-platinum (FePt) nanoparticles.

Suitably, the radionuclide is a positron emitter.

In one embodiment, the positron emitter is selected from the group consisting of copper (⁶⁰Cu, ⁶¹Cu, ⁶⁴Cu), scandium (⁴⁴Sc), titanium (⁴⁵Ti), iron (⁵²Fe), manganese (⁵¹Mn, ⁵²Mn), cobalt (⁵⁵Co), gallium (⁶⁶Ga, ⁶⁸Ga), arsenic (⁷²As), rubidium (^{82m}Rb), strontium (⁸³Sr), technetium (^{94m}Tc), yttrium (⁸⁶Y), zirconium (⁸⁹Zr) and indium (¹¹⁰In, ¹¹¹In).

Preferably, the positron emitter is ⁶⁴Cu.

In one embodiment, the inner layer is formed from a species selected from the group consisting of a metal, a metal oxide, a metal chalcogenide, a metal pnictogenide, a metalloid, a metalloid oxide, a metalloid chalcogenide, a metalloid pnictogenide, a self-assembled monolayer, a polymer, a protein, a carbohydrate and a biopolymer.

Preferably, the inner layer is formed from silica (SiO₂).

Preferably, the multimodal PET/MRI contrast agent further comprises at least one fluorophore.

In one embodiment, the at least one fluorophore is coupled to a surface of the second layer.

In one embodiment, the at least one fluorophore is embedded substantially within the inner layer.

Preferably, the multimodal PET/MRI contrast agent further comprises at least one quantum dot.

In one embodiment, the at least one quantum dot is associated with at least one magnetic material to form said magnetic signal generating core.

In one embodiment, the at least one quantum dot is coupled to a surface of the outer layer.

In one embodiment, the at least one quantum dot is embedded substantially within the inner layer.

According to a second aspect of the present invention there is provided a process of synthesizing a multimodal PET (positron emission tomography)/MRI (magnetic resonance imaging) contrast agent comprising:
contacting a surface of a magnetic signal generating core with a functionalizing solution to form an inner layer at least partially over said magnetic signal generating core to define a functionalized surface;
contacting said functionalized surface with a sensitizing solution containing at least a source of bivalent tin ions to form a tin-sensitized surface;
contacting said tin-sensitized surface with an activator solution containing at least a source of palladium ions to form a palladium-activated surface; and
contacting said palladium-activated surface with an electroless plating solution containing at least a source of radionuclide ions and a reducing and/or hydrolyzing agent for treating said radionuclide ions to form an electrolessly plated layer of said radionuclide on the palladium-activated surface.

In one embodiment, the reducing agent is formaldehyde.

In one embodiment, the hydrolyzing agent is an alkali selected from the group consisting of ammonia, lithium hydroxide, sodium hydroxide, potassium hydroxide and ammonium hydroxide.

In one embodiment, the functionalizing solution comprises a tetraethyl orthosilicate (TEOS) precursor, the process further comprising:
polymerizing the TEOS precursor to form the inner layer as a silica (SiO₂) shell that at least partially encapsulates the magnetic signal generating core.

Suitably, the step of forming the inner layer to define the functionalizing surface occurs at a pH value that falls within a range of 6 to 14.

In one embodiment, the sensitizing solution comprises tin (II) chloride (SnCl₂) as a source of bivalent tin ions.

Suitably, the step of forming the tin-sensitized surface occurs at a pH value that falls within a range of 1 to 8.

In one embodiment, the activator solution comprises palladium nitrate as a source of palladium ions.

Suitably the step of forming the precious metal-activated surface occurs at a pH value that falls within a range of 1 to 13.

Suitably, the radionuclide is a positron emitter.

In a preferred embodiment, the positron emitter is ⁶⁴Cu.

In one embodiment, the step of forming electroless plating layer occurs at a pH value that falls within a range of 1 to 13.

In one embodiment, the electroless plating solution additionally comprises at least one additive selected from the group consisting of stabilizers, complexing agents and surfactants.

According to a third aspect of the present invention there is provided a multimodal PET (positron emission tomography)/MRI (magnetic resonance imaging) contrast agent synthesized by a process according to the second aspect.

According to a fourth aspect of the present invention there is provided a pharmaceutical formulation comprising a multimodal PET (positron emission tomography)/MRI (magnetic resonance imaging) contrast agent as claimed in any one of claims 1 to 16, and a pharmaceutically acceptable excipient, wherein the formulation is suitable for administration to a patient as a multimodal PET/MRI contrast agent, and wherein the multimodal PET/MRI contrast agent is present in the formulation in an amount sufficient to enhance one or more of the following images: a magnetic resonance imaging (MRI) image, a positron emission tomography (PET) image, a single photon emission computed tomography (SPECT) image and a computed tomography (CT) image.

In one embodiment, the pharmaceutically acceptable excipient is a buffered saline.

In one embodiment, the multimodal PET/MRI contrast agent is present in the formulation in an amount ranging from about 0.0001% to about 25% by weight based on the total weight of the formulation.

According to a fifth aspect of the present invention there is provided a method of imaging a region of interest in a patient using multimodal PET/MRI imaging, the method comprising:
administering to a patient a pharmaceutical formulation according to the fourth aspect;
detecting arrival of the multimodal PET/MRI contrast agent present in the formulation in the region of interest by comparing the PET and/or MRI signals in said region of interest during or after administering said formulation;
collecting PET and MRI image data of the region of interest; and
constructing a multimodal PET/MRI image of said region of interest using the PET and MRI image data, wherein the region of interest appears distinct from the background tissue.

In one embodiment, the formulation is administered to the patient by intramuscular or intravenous injection.

Suitably, the patient is one of a human or non-human subject.

According to a sixth aspect of the present invention there is provided a use of a multimodal PET (positron emission tomography)/MRI (magnetic resonance imaging) contrast agent according to the first aspect in the preparation of a pharmaceutical formulation for imaging a region of interest in a patient using multimodal PET/MRI imaging.

Other aspects of the invention are also disclosed.

### Brief Description of Drawings

Preferred embodiments of the invention will now be described, by way of example only, with reference to the accompanying drawings in which:
**Figures 1** to **3** show transmission electron microscopy (TEM) images of core shell nanoparticles produced according to a preferred embodiment of the present invention, comprising an iron oxide core and a first shell layer of silica encapsulating the iron oxide core, in which the silica layer has a thickness of 3 nm (Fig. 1), 5 nm (Fig. 2) and 8 nm (Fig. 3); scale: 50 nm;
**Figure 4** shows a TEM image of a plurality of monodispersed iron oxide nanoparticles produced according to a literature method modified accordingly for use in producing the core shell nanoparticles of the preferred embodiment of the present invention;
**Figure 5** shows an MRI signal intensity analysis of MRI phantoms obtained for a sample set (**2-6**) of core shell nanoparticles containing varying amounts of iron oxide corresponding to 0.11, 0.5, 1.0, 10 and 25 µg iron equivalent concentrations, sample (**1**) is a control sample (water);
**Figure 6** shows a high resolution transmission electron microscopy (HRTEM) image of core shell nanoparticles produced according to a preferred embodiment of the present invention, in which a first shell layer of silica encapsulating an iron oxide core of said core shell nanoparticles has been sensitized with bivalent tin ions; arrows represent tin clusters;
**Figure 7** shows a series of energy-dispersive X-ray (EDX) analysis images mapping the elements (iron, oxygen, tin and silicon) present in the tin-sensitized core shell nanoparticles of Fig. 6, together with a contrast image;
**Figure 8** shows an EDX spectrum taken of the tin-sensitized core shell nanoparticles of Fig. 7, with peaks corresponding to oxygen (0.525 keV), iron (0.705 and 6.404 keV), silicon (1.74 keV) and tin (3.444 keV);
**Figure 9** shows an MRI signal intensity analysis of MRI phantoms obtained for a sample set (**2-6**) of tin-sensitized core shell nanoparticles of Fig. 6, comprising a first shell layer of silica of thickness of about 1 nm, containing varying amounts of iron oxide corresponding to 0.11, 0.5, 1.0, 10 and 25 µg iron equivalent concentrations, sample (**1**) is a control sample (water);
**Figures 10** and **11** show HRTEM images of the core shell nanoparticles of Fig. 6 after the sensitized first shell layer of silica has been subsequently seeded with palladium ions; arrows represent palladium clusters;
**Figure 12** shows an HRTEM image of the core shell nanoparticles of Figs. 10 and 11 after the palladium seeded first shell layer of silica has been electrolessly plated with non-radioactive copper;
**Figure 13** shows a plot of temperature-dependent loading of non-radioactive copper electrolessly plated on the palladium seeded first shell layer of silica on the core shell nanoparticles of Figs. 10 and 11;
**Figure 14** shows a series of EDX analysis images mapping the elements (iron, oxygen, tin, silicon, palladium and copper) present in the electrolessly copper plated core shell nanoparticles of Fig. 12, together with a contrast image;
**Figure 15** shows an EDX spectrum taken of the electrolessly copper plated core shell nanoparticles of Fig. 12, with peaks corresponding to oxygen (0.525 keV), iron (0.705 and 6.404 keV), silicon (1.74 keV), tin (3.444 keV) and copper (0.923 and 8.048 keV), together with a peak (7.478 keV) associated with the nickel substrate upon which the measurements were taken;
**Figure 16** shows an MRI signal intensity analysis of MRI phantoms obtained for a sample set (**2-5**) of the electrolessly copper plated core shell nanoparticles of Fig. 12, containing varying amounts of iron oxide corresponding to 0.11, 0.5, 1.0 and 10 µg iron equivalent concentrations, sample (**1**) is a control sample (water);
**Figure 17** shows an HRTEM image of the core shell nanoparticles of Figs. 10 and 11 after the palladium seeded first shell layer of silica has been electrolessly plated with radioactive copper (⁶⁴Cu); arrows represent radioactive ⁶⁴Cu converted to nickel after radioactive decay;
**Figure 18** shows a plot of temperature-dependent loading of radioactive copper electrolessly plated on the palladium seeded first shell layer of silica on the core shell nanoparticles of Figs. 10 and 11;
**Figure 19** shows a series of EDX analysis images mapping the elements (iron, oxygen, tin, silicon, palladium and copper) present in the electrolessly radioactive copper (⁶⁴Cu) plated core shell nanoparticles of Fig. 17, together with a contrast image;
**Figure 20** shows an EDX spectrum taken of the electrolessly copper (⁶⁴Cu) plated core shell nanoparticles of Fig. 17, with peaks corresponding to oxygen (0.525 keV), iron (0.705 and 6.404 keV), silicon (1.74 keV) and tin (3.444 keV) together with a peak (0.923 keV) associated with the copper substrate upon which the measurements were taken and another peak (7.478 keV) associated with nickel which is produced following radioactive decay of ⁶⁴Cu;
**Figures 21** and **22** show Radio-instant thin layer chromatography (Radio-ITLC) chromatograms obtained for a ⁶⁴Cu control and the electrolessly copper (⁶⁴Cu) plated core shell nanoparticles of Fig. 17, when conducted in 1 ml of 10 mM phosphate buffered saline (PBS) containing 10% wt/vol EDTA;
**Figure 23** shows a multimodal PET/MRI image (obtained after 18 hours) of a six week old female mouse (C57BL/6) that has been injected through the tail vein with the electrolessly copper (⁶⁴Cu) plated core shell nanoparticles of Fig. 17;
**Figure 24** shows the corresponding MRI portion of the multimodal PET/MRI image of Fig. 23;
**Figure 25** shows a multimodal PET/MRI image (obtained after 15 minutes) of a second six week old female mouse (C57BL/6) that has been injected through the tail vein with the electrolessly copper (⁶⁴Cu) plated core shell nanoparticles of Fig. 17 (aged for two days);
**Figure 26** shows a TEM image of the core shell nanoparticles of Figs. 10 and 11 after the palladium seeded first shell layer of silica has been electrolessly plated with non-radioactive indium;
**Figure 27** shows a series of EDX analysis images mapping the elements (iron, oxygen, silicon and indium) present in the electrolessly indium plated core shell nanoparticles of Fig. 26, together with a contrast image;
**Figure 28** shows an EDX spectrum taken of the electrolessly indium plated core shell nanoparticles of Fig. 26, with peaks corresponding to oxygen (0.525 keV), iron (0.705 and 6.404 keV), silicon (1.74 keV), tin (3.444 keV) and indium (3.286 keV), together with a peak (0.923 keV) associated with the copper substrate upon which the measurements were taken;
**Figure 29** shows a TEM image of the core shell nanoparticles of Figs. 10 and 11 after the palladium seeded first shell layer of silica has been electrolessly plated with non-radioactive yttrium;
**Figure 30** shows a series of EDX analysis images mapping the elements (iron, oxygen, silicon and indium) present in the electrolessly yttrium plated core shell nanoparticles of Fig. 29, together with a contrast image;
**Figure 31** shows an EDX spectrum taken of the electrolessly yttrium plated core shell nanoparticles of Fig. 29, with peaks corresponding to oxygen (0.525 keV), iron (0.705 keV), silicon (1.74 keV) and yttrium (1.922 keV), together with a peak (0.923 keV) associated with the copper substrate upon which the measurements were taken;
**Figure 32** shows a TEM image of the core shell nanoparticles of Figs. 10 and 11 after the palladium seeded first shell layer of silica has been electrolessly plated with non-radioactive zirconium;
**Figure 33** shows a series of EDX analysis images mapping the elements (iron, oxygen, silicon and indium) present in the electrolessly zirconium plated core shell nanoparticles of Fig. 32, together with a contrast image; and
**Figure 34** shows an EDX spectrum taken of the electrolessly zirconium plated core shell nanoparticles of Fig. 32, with peaks corresponding to oxygen (0.525 keV), iron (0.705 keV), silicon (1.74 keV) and zirconium (2.304 keV), together with a peak (0.923 keV) associated with the copper substrate upon which the measurements were taken.

### Detailed Description

It is to be understood that the following description is for the purpose of describing particular embodiments only.

The present invention is predicated on the finding of a process for synthesizing multimodal PET (positron emission tomography)/MRI (magnetic resonance imaging) contrast agents suitable for enhancing the contrast of images obtained during PET/MRI imaging that does away with the need for a chelator or complexing agent to bind the radionuclide responsible for providing the PET signal.

Moreover, the process and the multimodal PET/MRI contrast agents synthesized according to this presently claimed process, represent a significant departure from the chelator-free contrast agents that have previously been developed for the purpose of medical imaging. For instance, the radiolabelled ferrite particles described in US Patent Application No. 2004/0081617 A1 (in the name of Browitt et al.)^{[1]} are obtained via the co-precipitation method, which is notably limited by the fact that the particles resulting from this method are polydispersed with sizes ranging from 5 nm to 200 nm. This limitation not only impacts upon the magnetic properties of the obtained particles (batch to batch variation), but also raises doubts as to the stability, biocompatibility, surface modification and overall suitability of these particles for applications *in vivo.*

### Process

A process of synthesizing a multimodal PET (positron emission tomography)/MRI (magnetic resonance imaging) contrast agent according to a preferred embodiment of the present invention will now be described.

In its simplest form, the process comprises steps to modify a magnetic signal generating core by applying a coating portion in the form of a plurality of layers, in which an inner layer presents a plurality of suitably functionalised surface groups that can be activated to promote the formation of a second layer of an electrolessly plated radionuclide metal around the magnetic signal generating core.

### Magnetic Signal Generating Core

As will be appreciated by those skilled in the relevant art, the core of a contrast agent to be used for MRI imaging purposes can be either a ferromagnetic, paramagnetic or superparamagnetic signal generating core. Ferromagnetic materials generally contain iron (Fe), cobalt (Co) or nickel (Ni). Such materials have a large positive magnetic susceptibility when placed in an external magnet field, and have the ability to remain magnetized when the external magnetic field is removed, which is a distinguishing factor when compared to paramagnetic, superparamagnetic, and diamagnetic materials. Paramagnetic materials include oxygen and ions of various metals like iron (Fe) and gadolinium (Gd) for example. These ions have unpaired electrons, resulting in a positive magnetic susceptibility. The magnitude of this susceptibility is significantly less than that of ferromagnetic materials. Superparamagnetic materials on the other hand, consist of individual domains of elements that have ferromagnetic properties in bulk. Their magnetic susceptibility is between that of ferromagnetic and paramagnetic materials.

Thus, for the purposes of the present invention, the magnetic signal generating core of the multimodal PET/MRI contrast agent comprises at least one metal selected from the group consisting of iron (Fe), cobalt (Co), nickel (Ni), neodymium (Nd), gadolinium (Gd) and manganese (Mn).

In one embodiment, the magnetic signal generating core may take the form of an oxide, a mixed oxide and/or a hydroxide of at least one of Fe, Co, Ni, Nd, Gd and Mn. For example, in the case of iron (Fe), the magnetic signal generating core may be produced from an iron oxide selected from the group consisting of magnetite (Fe₃O₄), maghemite (γ-Fe₂O₃) and hematite (α-Fe₂O₃), or a mixture thereof. In the case of gadolinium (Gd), the magnetic signal generating core may take the form of gadolinium hydroxide (Gd(OH)₃) nanoparticles. While the case of manganese (Mn), the magnetic signal generating core may take the form of magnesium-based layered double hydroxide (Mn-LDH) nanoparticles.

In another embodiment, the magnetic signal generating core may take the form of an alloy of one or more of these metals (Fe, Co, Ni, Nd, Gd and Mn), potentially in combination with another metal. Alloy-based nanomaterials are described as good candidates for developing T₂ contrast agents with higher relaxivities.

In one embodiment, the magnetic signal generating core may take the form of iron-based alloy nanoparticles, such as iron-cobalt (FeCo) and iron-platinum (FePt) nanoparticles. FePt nanoparticles are chemically more stable than Fe and FeCo nanoparticles, and have been shown to have great potential as contrast agents for MRI and computed tomography (CT) imaging. Other iron-based alloy nanoparticles that may find application as a magnetic signal generating core include gold-iron oxide (Au-Fe₃O₄) nanoparticles.

The substitution of one of the Fe ions in an iron oxide for a different magnetic atom (Mn, Zn, Co, Ni) produces compounds known as ferrites. Such ferrite materials are characterized by their high saturation magnetization, which increases the relaxation rate.

In another embodiment, the magnetic signal generating core of the multimodal PET/MRI contrast agent may take the form of a ferrite material with a spinel-, garnet-, magnetoplumbite-, or other hexagonal structure, such as those disclosed in US Patent Application No. 2007/0258888 A1 (in the name of Feldmann et al.)^{[3]}.

In another embodiment, the magnetic signal generating core may take the form of a support material loaded with at least one metal ion selected from the group consisting of iron (Fe), cobalt (Co), nickel (Ni), neodymium (Nd), gadolinium (Gd) and manganese (Mn).

In the magnetic signal generating core of the multimodal PET/MRI contrast agent, it is possible to integrate other materials within the core, without impacting negatively on the magnetic properties of the magnetic material.

In one embodiment, the magnetic signal generating core further includes one or more quantum dots to introduce fluorescence imaging as a further mode of imaging, which is complementary to both PET and MRI imaging.

In a preferred embodiment, the inventors have obtained good results when the magnetic signal generating core is formed from iron oxide nanoparticles.

### Coating Portion

As indicated above, the coating portion used to coat the magnetic signal generating core includes an inner layer that presents a plurality of suitably functionalised surface groups that can be activated to promote the formation of a second layer of an electrolessly plated radionuclide metal around the magnetic signal generating core.

An inner layer suitable for coating the magnetic signal generating core of the multimodal PET/MRI contrast agent and provide the surface groups necessary for further activation may take one of several forms.

For instance, in one embodiment, the inner layer may take the form of a self-assembled monolayer (SAM) of a plurality of long chain ligands suitably functionalised with groups capable of undergoing reaction with the groups on the surface of the magnetic core. For example, where the magnetic core comprises iron oxide (Fe₃O₄) nanoparticles, it is possible to modify the surface of the iron oxide particles by silanisation with ligands having a trialkoxy(alkyl)silyl moiety at one end. It will be appreciated that once the SAM has been formed on the surface of the iron oxide particles, the other end of the long chain ligands presents a new surface group that is itself suitably functionalised to be activated to promote the formation of the electrolessly plated radionuclide metal layer, or can be further modified so that it becomes suitably functionalised.

In one embodiment, the inner layer may take the form of a polymer that is suitably functionalised with groups capable of undergoing reaction with the groups on the surface of the magnetic core. For example, where the magnetic core comprises iron oxide (Fe₃O₄) nanoparticles, it is possible to modify the surface of the iron oxide particles by grafting with suitably terminated polymer chains. Again, it will be appreciated that once the polymer chains have been grafted on the surface of the iron oxide particles, the other end of the polymer chains presents a new surface group that is itself suitably functionalised to be activated to promote the formation of the electrolessly plated radionuclide metal layer, or can be further modified so that it becomes suitably functionalised.

In one embodiment the inner layer may take the form of a shell layer that partially or wholly encapsulates the magnetic signal generating core to realise a core-shell type arrangement. Suitable shells may include metal or metalloid oxide shells such as the oxides, chalcogenides or pnictogenides formed from silicon, titanium, zinc, tin or aluminium.

In the case of silica (SiO₂), it will be appreciated by those skilled in the relevant art, that the formation of a silica shell around a central core can be implemented by a number of methods including the Stöber method, reverse emulsion, and the like.

In other embodiments, the inner layer may be formed from another species entirely, such as those selected from the group consisting of a polyelectrolyte, protein, a carbohydrate or biopolymer. Examples may include poly(allylamine hydrochloride), poly(glutamic acid), poly-lysine, poly-styrene and chitosan.

In a preferred embodiment, the inventors have obtained good results when the inner layer takes the form of a silica (SiO₂) shell layer to encapsulate the iron oxide nanoparticles to realise a core-shell type arrangement. Specifically, the silica (SiO₂) shell layer is formed using the reverse emulsion method by polymerizing a solution (pH in the range of about 6 to about 14) of tetraethyl orthosilicate (TEOS) as precursor to define a functionalized surface suitable for promoting electroless deposition.

### Electroless Plating

In general terms, the process of electroless plating requires the use a redox reaction to deposit metal onto a surface of the substrate to be plated without the passage of an electric current. The reaction typically involves the use of a suitable catalyst to facilitate the reduction process of the reaction. The most commonly used catalyst system employs the use of palladium chloride (PdCl₂)/ tin (II) chloride (SnCl₂) catalyst composition in acidic (HCI) solution. Specifically, the substrate surface is first sensitized by immersion in the tin (II) chloride solution, followed by activation in the palladium chloride solution. The activated surface is then exposed to a solution of the metal to be plated.

The inventors have obtained good results when the electroless plating step of the process is conducted by contacting said functionalized surface of the silica (SiO₂) shell layer encapsulating the iron oxide nanoparticle core with a sensitizing solution (pH in the range of about 1 to about 8) containing Sn²⁺ ions using tin (II) chloride (SnCl₂) as a source of said bivalent tin ions to form a tin-sensitized surface, contacting the tin-sensitized surface with an activator solution (pH in the range of about 1 to about 13) containing Pd²⁺ ions using a palladium nitrate solution as a source of said palladium ions, which are subsequently reduced, thereby realising a palladium-activated surface consisting of metallic Pd⁰ nuclei. The palladium-activated surface is then contacted by an electroless plating solution (pH in the range of about 1 to about 13) containing ions of the radionuclide metal to be plated and a reducing agent to reduce the radionuclide metal ions to form an electrolessly plated layer of the radionuclide metal on the palladium-activated surface of the silica (SiO₂) shell layer.

In other embodiments, rather than using a reducing agent, the radionuclide metal may be hydrolyzed to generate the radionuclide metal ions. For instance, the hydrolyzing agent may be an alkali selected from the group consisting of ammonia, lithium hydroxide, sodium hydroxide, potassium hydroxide and ammonium hydroxide.

It will be appreciated by those skilled in the relevant art that the electroless plating solution may additionally comprise one or more additives selected from the group consisting of stabilizers, complexing agents and surfactants.

### Radionuclide

For the purposes of the present invention, it will be understood that the radionuclide metal used for realising PET imaging must be a positron emitter with a half-life that is sufficiently long enough to enable a reasonable PET image to be produced. As such, suitable positron emitters may be selected from the group consisting of copper (⁶⁰Cu, ⁶¹Cu, ⁶⁴Cu), scandium (⁴⁴Sc), titanium (⁴⁵Ti), iron (⁵²Fe), manganese (⁵¹Mn, ⁵²Mn), cobalt (⁵⁵Co), gallium (⁶⁶Ga, ⁶⁸Ga), arsenic (⁷²As), rubidium (^{82m}Rb), strontium (⁸³Sr), technetium (^{94m}Tc), yttrium (⁸⁶Y), zirconium (⁸⁹Zr) and indium (¹¹⁰In).

In a preferred embodiment, the inventors have obtained good results when the positron emitter is ⁶⁴Cu with a concentration in the electroless plating solution in the range of between about femtomolar to several tens of Molar. The reducing agent used in this instance is formaldehyde having an equivalent concentration in the range of between about femtomolar to several tens of Molar.

By virtue of the above process, it is possible to bind such radionuclides directly to the surface of the silica (SiO₂) shell layer encapsulating the iron oxide nanoparticle core, thereby removing the need for a complexing agent or chelator.

In other embodiments, the hydrolyzing agent is an alkali selected from the group consisting of ammonia, lithium hydroxide, sodium hydroxide, potassium hydroxide and ammonium hydroxide.

### Multimodal PET/MRI Contrast Agent

For the purpose of demonstrating one particular preferred embodiment of the present invention, the process for synthesizing multimodal PET/MRI contrast agents will be described in terms of a core-shell nanoparticle arrangement using iron oxide nanoparticles as the magnetic signal generating core, with a coating portion that includes a silica shell inner layer that presents a plurality of silanol groups on the surface of the shell for use in facilitating the formation of an electrolessly plated radionuclide metal outer layer.

### Iron oxide nanoparticles

According to a first step (**A**) of the process, nanoparticles having a superparamagnetic iron oxide core were synthesized via a two-stage process. In the first stage, iron oxide nanoparticles were produced by thermal decomposition according to a literature method (Park *et al.*, 2004)^{[2]}. In the second stage, the iron oxide nanoparticles obtained via the thermal decomposition method were then purified using a cleaning protocol developed by the present inventors and which forms the subject of International PCT patent application no. PCT/AU2017/050981 (Gammilonghi et al.)^{[4]}*.* In this respect, the magnetic nanoparticles were purified by washing in a first solvent composition comprising a 1:1 (vol/vol) ratio of diethyl ether and methanol, followed by washing in a second solvent composition comprising a 1:1 (vol/vol) ratio of hexane and ethanol, before finally being dispersed in a third solvent composition comprised of cyclohexane.

### Silica-coated iron oxide nanoparticles

According to a second step (**B**), a silica (SiO₂) shell is grown around the purified iron oxide nanoparticles in order to render the nanoparticles dispersible in polar solvents such as water. The formation of the SiO₂ shell on the surface of the purified iron oxide nanoparticles was performed through the formation of a water in-cyclohexane reverse microemulsion according to a literature method (Han *et al.*, 2008)^{[5]}, but which has been modified according to a protocol which will now be described. Specifically, the purified iron oxide nanoparticles were first well-dispersed in a cyclohexane solution comprising a suitable surfactant to improve conditions for hydrophilicity. The solution of well-dispersed nanoparticles was then subsequently mixed with tetraethyl orthosilicate (TEOS) in a 1:1 (vol/vol) ratio for a few minutes, followed by the addition of ammonia solution (28 wt. % in water) to form a reverse microemulsion. Silica-coated iron oxide nanoparticles with an overall particle size of about 15 nm (± 1 nm) were obtained after 6 hours of reaction under stirring at room temperature. Once the reaction was completed, methanol was added to disrupt the reverse microemulsion, and the silica-coated iron oxide nanoparticles were extracted from the methanol phase using magnetic separation or centrifugation at room temperature.

The inventors have found that the silica-coated purified iron oxide nanoparticles obtained according to the above protocol are readily dispersible in polar solvents such as water, methanol, or ethanol, and the like.

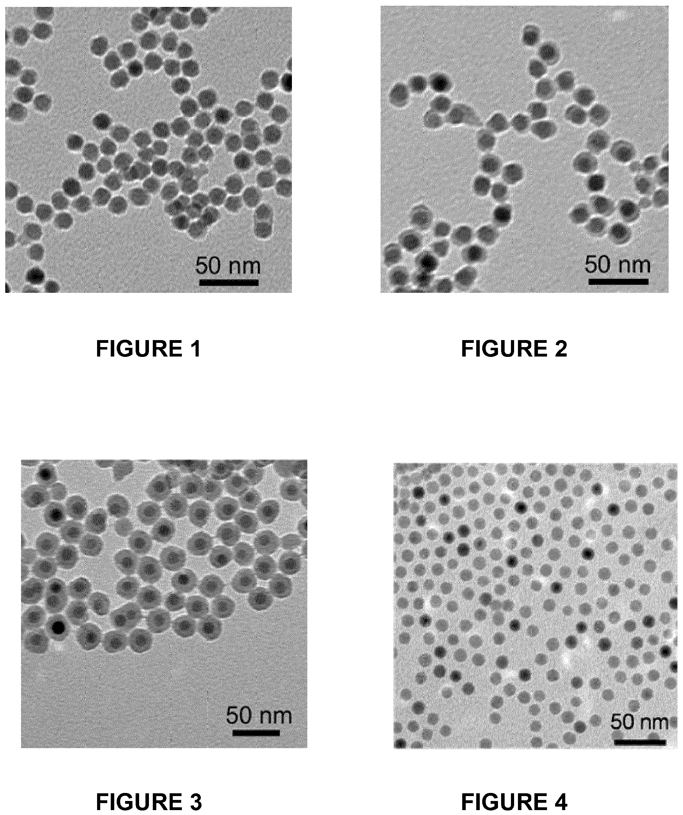

Referring specifically to **Figures 1-3****,** the inventors have found that it is possible to alter the thickness of the silica shell surrounding the iron oxide nanoparticles by altering the amount of TEOS that is added to the solution. For instance, by changing the amount of TEOS from the 20 µL used above in the 1:1 (vol/vol) ratio of purified iron oxide nanoparticles to TEOS to 30 µL, 40 µL or 50 µL it is possible to increase the thickness of the silica shell from 1 nm to 3 nm, 5 nm or 8 nm, respectively. The inventors have also found that variations in the stirring speed, concentration of purified iron oxide nanoparticles and temperature all played an important role in the synthesis and even minor variations were observed to impact on the monodispersity of the obtained silica-coated iron oxide nanoparticles and the reproducibility of the core shell structures.

As shown in the TEM image in **Figure 4****,** the iron oxide nanoparticles produced via the above detailed methodology appear monodispersed, 12 nm (±1 nm) in diameter and spherical in morphology.

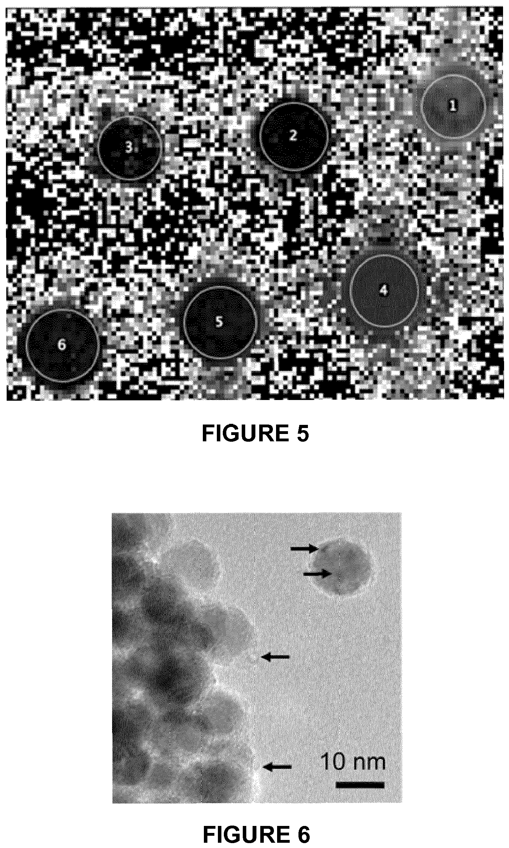

**Figure 5** shows the results of an MRI signal intensity analysis of the MRI phantoms obtained for the purified iron oxide nanoparticles conducted using a 9.4 Tesla Bruker Biospec MRI scanner. Specifically, sample (**1**) shows no shortening of the T2 signal as it is a control sample (water). Samples (**2 -6**) on the other hand, contain increasing amounts of nanoparticles corresponding to 0.11 µg, 0.5 µg, 1.0 µg, 10 µg and 25 µg iron equivalent concentrations. The results for samples (**2-6**) show that from the observed MRI phantoms, the purified iron oxide nanoparticles give a calculated T2 value of 196.9 mol⁻¹ at 9.4 Tesla. These results also show that as the concentration of nanoparticles is gradually increased, there is an increased shortening of the T2 signal and a proportional darkening of each associated MRI phantom. This demonstrates a decreased signal intensity (darkening effect) with increasing iron oxide concentration.

### Tin-sensitized silica-coated iron oxide nanoparticles

To coat the water-dispersible, silica-coated nanoparticle core with a radionuclide requires a three-step '*sensitization-seeding-deposition'* process adapted from the Applicant's own work (Anderson *et al.*, 2016)^{[6]}.

Thus, according to a third step (**C**), the silica-coated iron oxide nanoparticles obtained from (**B**) are first dispersed in water. A solution containing 5 mg equivalent of iron is subsequently withdrawn and centrifuged at 10,000 RPM for around 5 minutes to pelletize the nanoparticles. Following removal of the supernatant, the resulting pellet is added to an acidic solution of tin chloride (SnCl₂), where it is then mixed and allowed to react for approximately 10 minutes to allow the positive Sn²⁺ ions to bind to the negatively-charged silica surface. The resulting tin-sensitized silica-coated iron oxide nanoparticles are centrifuged at 10,000 RPM for 5 minutes, followed by rinsing and centrifuging with deionised water to remove any unbound Sn²⁺ ions.

**Figure 6** shows a TEM image of the tin-sensitized silica-coated iron oxide nanoparticles. The TEM image confirms that these particles have a thin silica shell of approximately 1 nm around a 12 nm iron oxide core. Upon closer examination of **Figure 6**, small lumps appear to be present on the surface of the silica-coated nanoparticles (as indicated by arrows) where tin sensitization has occurred. These small lumps appear as dark spots embedded in the silica shell of the nanoparticles. These dark spots are unique to the tin sensitised nanoparticles and do not appear in any of the prior TEM images described above.

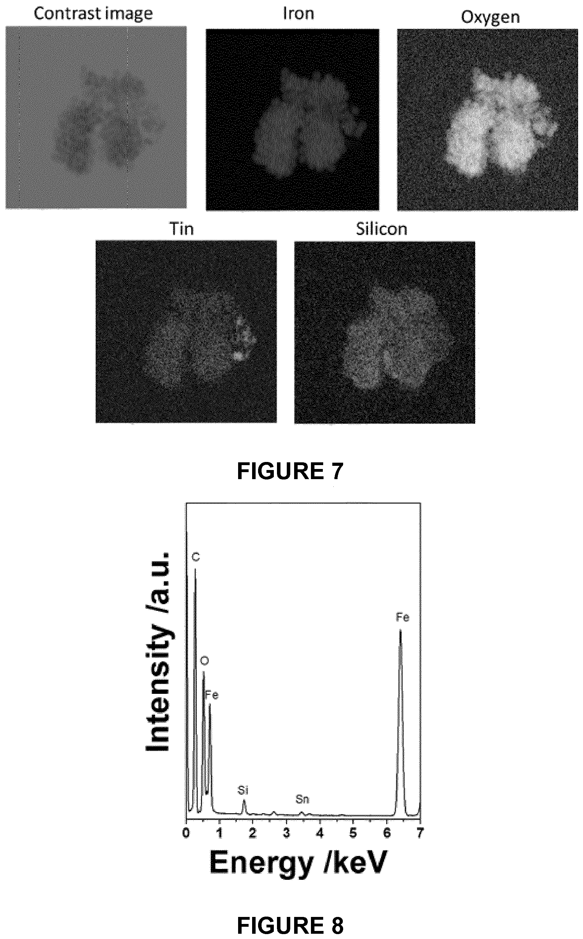

**Figure 7** shows a series of energy-dispersive X-ray (EDX) analysis images mapping the elements (iron, oxygen, tin and silicon) present in the tin-sensitized core shell nanoparticles of **Figure 6**, together with a contrast image, which confirms the presence of tin in the particles.

**Figure 8** shows the corresponding EDX spectra for the elemental mapping in **Figure 7**, with peaks corresponding to oxygen (0.525 keV), iron (0.705 and 6.404 keV) silicon (1.74 keV) and tin (3.444 keV) evident. Furthermore, ICP-MS analysis (not shown) of these tin-sensitized silica-coated iron oxide nanoparticles post-digestion in concentrated nitric acid further reveals that the particles have the following composition: 1 molar equivalent of Sn per 144 molar equivalents of Fe.

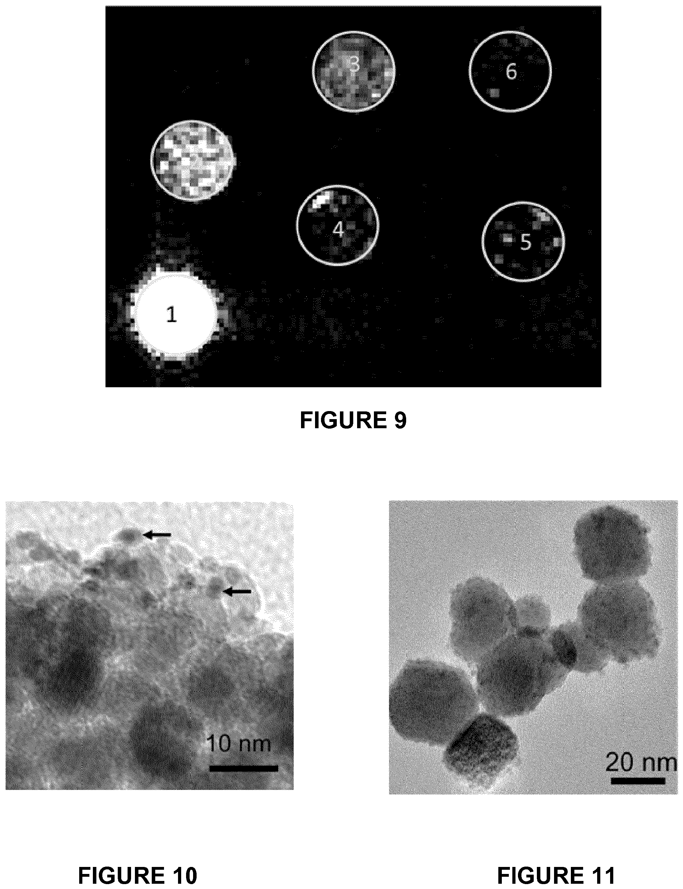

**Figure 9** shows the results of an MRI signal intensity analysis of the MRI phantoms obtained for the tin-sensitized silica-coated iron oxide nanoparticles conducted using a 9.4 Tesla Bruker Biospec MRI scanner. Specifically, sample (**1**) shows no shortening of the T2 signal as it is simply a control (water) completely void of any iron. Samples (**2 -6**) on the other hand, contain increasing amounts of the iron oxide nanoparticles, corresponding to 0.11 µg, 0.5 µg, 1.0 µg, 10 µg and 25 µg iron equivalent concentrations. The results for samples (**2-6**) show that from the observed MRI phantoms, the tin-sensitized silica-coated iron oxide nanoparticles give a calculated T2 value of 114.9 mol⁻¹ at 9.4 Tesla. When compared to the T2 value (196.9 mol⁻1) calculated for the purified iron oxide nanoparticles (see **Figure 5**), the impact of the silica shell appears to slightly reduce the magnetic capability of the iron oxide nanoparticle.

**Figure 9** thus shows a similar trend to that observed with respect to the purified iron oxide nanoparticles (see **Figure 5**) in that the shortening of the T2 signal (darkening effect) increases with increasing iron oxide concentration.

### Palladium-activated silica-coated iron oxide nanoparticles

According to a fourth step (**D**), the tin-sensitized particles are subsequently exposed to a palladium nitrate solution (Pd(NO₃)₂•2H₂O) for 5 minutes, leading to deposition of metallic Pd⁰ nuclei on the tin-sensitised silica surface while simultaneously displacing the previously bound Sn²⁺ ions. During this seeding reaction, the spontaneous formation of Pd⁰ nuclei or seeds proceeds *via* the formation of Sn⁴⁺ ions during the reduction of Pd²⁺ to Pd⁰. The favourable difference in the standard reduction potentials of Pd^{2+/0} (+0.99 V vs. standard hydrogen electrode - SHE) and Sn^{4+/2+} (+0.15 V vs. SHE) allows the reaction to proceed spontaneously (Anderson *et al.*, 2016)^{[6]}. The Pd⁰ layer formed on the silica surface of the iron oxide nanoparticles through the spontaneous reaction between the Sn⁴⁺ and Pd²⁺ ions acts as a catalyst, that is, the silica surface is activated for subsequent radiolabelling.

**Figure 10** shows a TEM image of the palladium-activated silica-coated iron oxide nanoparticles. The TEM image shows only minor changes in size and shape of the palladium-activated silica-coated iron oxide nanoparticles when compared to TEM images obtained for the tin-sensitized (see **Figure 6**), silica-coated (see **Figure 4**) and purified iron oxide nanoparticles (see **Figure 1**) described above.

**Figure 11** shows a high resolution TEM (HRTEM) image of the palladium-activated silica-coated iron oxide nanoparticles, which reveals large clusters (highlighted by arrows) that appear on the surface of the nanoparticles where seeding of the palladium has occurred. Such palladium clusters become more pronounced and the particle size remarkably increases, if the reaction is allowed to continue for longer durations (for instance, around 20 minutes).

An ICP-MS analysis (not shown) of the palladium-activated silica-coated iron oxide nanoparticles (5 minutes reaction) post-digestion in concentrated nitric acid reveals that the nanoparticles have the following composition: 114,411:12:1 molar equivalents of Fe, Sn and Pd, respectively. This result confirms the presence of palladium in the sample. Furthermore, the ICP-MS results indicate that with the introduction of palladium into the system, the amount of tin compared to iron in the sample is notably reduced. The inventors believe this to be the result of the replacement of tin on the nanoparticle surface with palladium.

Conveniently, once the silica-coated iron oxide nanoparticles undergo sensitization and seeding as elaborated in steps (**C**) and (**D**), the obtained palladium-activated nanoparticles can either be radiolabelled immediately for use in clinical administration or may be stored for radiolabelling at a later stage just prior to clinical administration.

In either case, the palladium-activated silica surface of the iron oxide nanoparticles is radiolabelled with a suitable radionuclide via the technique of electroless deposition as will now be described.

### Cold copper plating of palladium-activated silica-coated iron oxide nanoparticles

In order to optimise the copper plating process while minimising cost and radioactive (hot) waste generation, the copper plating bath was developed using non-radioactive (cold) copper as a model system. To minimise variation between this cold system and the radioactive ⁶⁴Cu, an acidified copper chloride [pH 1.0, 10-12 M] was initially used, as discussed in the current examples. This is because ⁶⁴Cu is produced in copper chloride form and is delivered in a hydrochloric acid solution of pH 1.0.

More specifically, and according to a fifth step (**E**), a solution of the palladium-activated silica-coated iron oxide nanoparticles from step (**D**) in water is subjected to magnetic separation to isolate a wet pellet containing the palladium-activated silica-coated iron oxide nanoparticles from the supernatant. Sodium hydroxide (or any other alkali such as potassium hydroxide) is added to the wet pellet, followed immediately by sodium potassium tartrate. In the subsequent step, a solution of the desired cold copper source is introduced. This is followed by the addition of the formaldehyde reducing agent. The order in which the reactants are added is significant. Furthermore, the rate of reaction can be controlled through temperature.

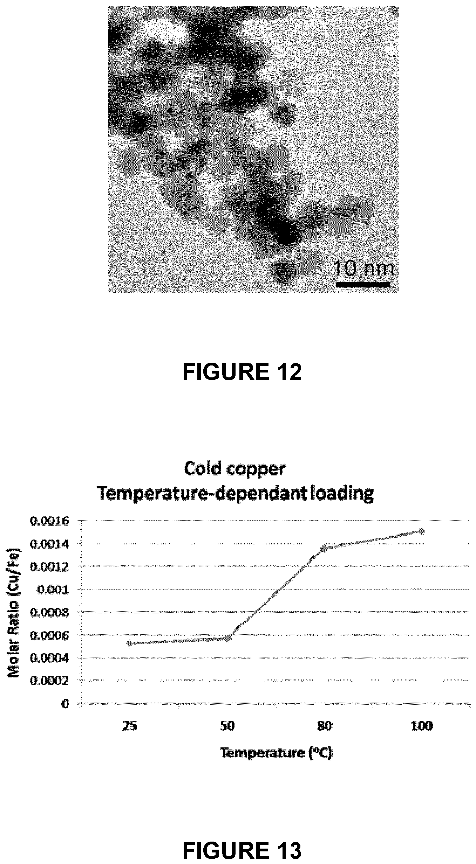

Since the reaction is performed in water, the optimisation was restricted to the sub-boiling point of the solvent, (specifically 80°C in the current case). However, **Figure 13** shows that a range of temperatures is possible to control the amount of copper loading onto the palladium-activated silica surface of the iron oxide nanoparticles. In the current examples, the reaction mixture is heated to 80°C for 45 minutes, but both time and temperature of the reaction can be potentially varied.

Once the reaction is complete, the resulting particles are rinsed twice with deionised water using magnetic separation or centrifugation to remove any remaining reactants or by-products. The resulting pellet is then dispersed in a suitable solvent (typically water, saline, buffer or biological growth media) through 2 minutes of sonication (other forms of mixings such as agitation, pipetting, vortexing for varying amounts of time are also possible) to obtain the electrolessly deposited copper plated iron oxide nanoparticles.

As shown in **Figure 12**, the TEM image of the non-radioactive electrolessly deposited copper plated iron oxide nanoparticles shows that the original particle size has now significantly increased to around 12 to 15 nm in diameter, indicating deposition of the non-radioactive copper onto the surface of the silica-coated iron oxide nanoparticles.

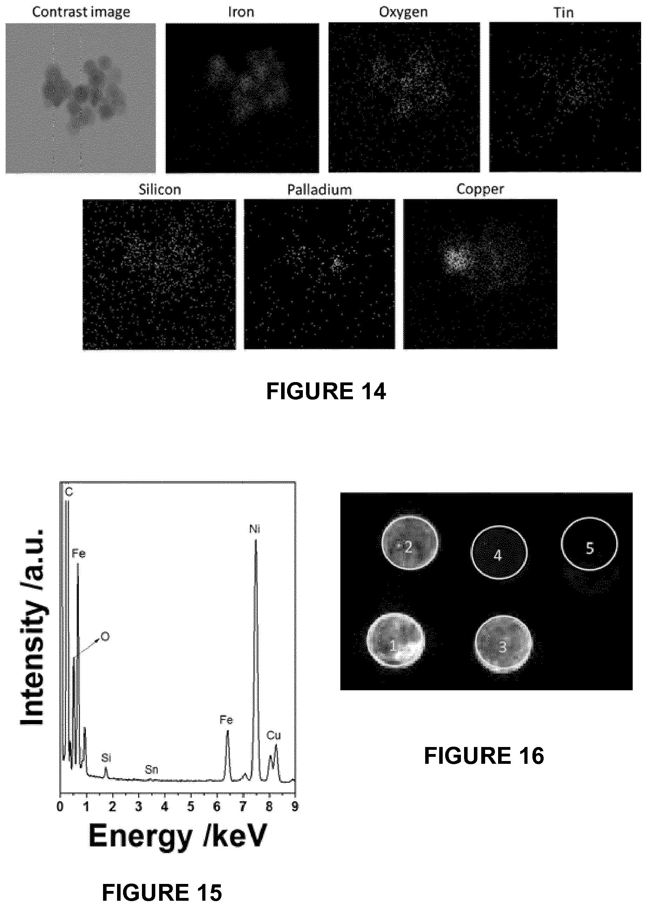

Elemental mapping of these non-radioactive electrolessly deposited copper plated iron oxide nanoparticles using EDX in **Figure 14** further confirms the presence of Cu, in addition to Fe, Si, O, Sn and Pd.

**Figure 15** shows the corresponding EDX spectrum of the non-radioactive electrolessly deposited copper plated silica-coated iron oxide nanoparticles. The EDX spectrum reveals peaks representative of oxygen (0.525 keV), iron (0.705 and 6.404 keV) and silicon (1.74 keV). Additional peaks for copper are observed at 8.048 and 0.923 keV confirming successful electroless deposition of the metal. The peak observed at 7.478 keV corresponds to the nickel-based TEM substrate upon which the non-radioactive electrolessly deposited copper plated iron oxide nanoparticles are supported during measurement.

**Figure 16** shows the results of an MRI signal intensity analysis of the MRI phantoms obtained for the non-radioactive electrolessly deposited copper plated silica-coated iron oxide nanoparticles conducted using a 9.4 Tesla Bruker Biospec MRI scanner. Specifically, sample (**1**) shows no shortening of the T2 signal as it is a control (water) with no iron present. Samples (**2-5**) on the other hand, contain increasing amounts of the iron oxide nanoparticles, corresponding to 0.11 µg, 0.5 µg, 1.0 µg and 10 µg iron equivalent concentrations. The results for samples (**2-5**) show that from the observed MRI phantoms, the non-radioactive electrolessly deposited copper plated iron oxide nanoparticles give a calculated T2 value of 136.9 mol⁻¹ at 9.4 Tesla.

When compared to the T2 value (196.9 mol⁻¹) calculated for the purified iron oxide nanoparticles (see **Figure 5**), and the T2 value (114.9 mol⁻¹) calculated for the silica-coated iron oxide nanoparticles (see **Figure 9**), the T2 value is much improved after copper loading. This may be the result of (i) a subsequent etching of the silica shell surface during the electroless deposited copper plating process that reduces the overall thickness of the silica shell on the iron oxide core nanoparticles, and/or (ii) an enhancement of the magnetic properties due to the magnetic nature of the copper species.

**Figure 16** thus shows a similar trend to that observed with respect to the purified iron oxide nanoparticles (see **Figure 5**) and the silica-coated iron oxide nanoparticles (see **Figure 9**), wherein the shortening of the T2 signal (darkening effect) increases with increasing iron oxide concentration.

### Radioactive ⁶⁴Cu plating of palladium-activated silica-coated iron oxide nanoparticles

Once the copper bath protocol had been optimised for cold copper, the electroless plating process was carried out using ⁶⁴Cu as the 'hot' copper source. This was achieved by following the same protocol as detailed above with the minor variation of using ⁶⁴Cu instead of cold copper. It is known that the concentration of copper ions in commercially obtained ⁶⁴Cu is of the order of femtomolars. However, this copper concentration varies from batch-to-batch. Typically, the known concentration of received commercial radioactive agents is defined as MegaBecquerels (MBq). Therefore, in the current example, a fixed MBq concentration of ⁶⁴Cu was employed. The commercially obtained ⁶⁴Cu was diluted to 80 MBq/mL.

The fifth step (**E**) of the presently claimed process was repeated this time using the radioactive (⁶⁴Cu) from the stock solution, and the reaction was carried out at 80°C for 45 minutes to afford radioactive electrolessly deposited copper plated silica-coated iron oxide nanoparticles.

It is known that ⁶⁴Cu has a half-life of 12.7 hours, typically decaying into nickel. Thus, these radioactive electrolessly deposited copper plated iron oxide nanoparticles were analysed after a week to allow the ⁶⁴Cu radioactivity to decay to safe handling levels.

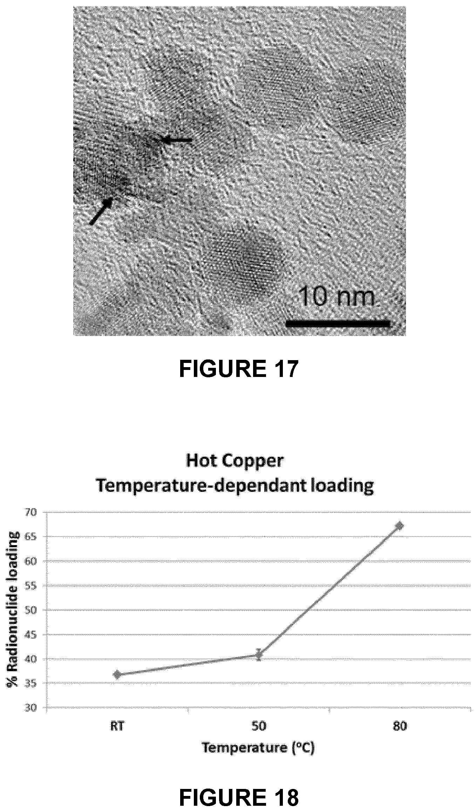

**Figure 17** shows an HRTEM image of the radioactive electrolessly copper plated iron oxide nanoparticles supported on a copper TEM substrate. The HRTEM image indicates the presence of small clusters/particles on the surface of the silica-coated iron oxide nanoparticles, while affirming that the size of radioactive electrolessly deposited copper plated iron oxide nanoparticles remains within a desirable 10 nm to 15 nm range. This diameter range is important considering that larger particle sizes may influence their biodistribution profile and clearance through the reticuloendothelial system (RES) *in vivo.*

There is a noticeable correlation between increasing reaction rate and increasing temperature, as reflected from the temperature-dependent loading data obtained for non-radioactive copper (**Figure 13****).** The corresponding temperature-dependent loading data obtained for the radioactive ⁶⁴Cu (**Figure 18**) also shows a significant increase in radioactive copper loading onto these nanoparticles with increase in temperature.

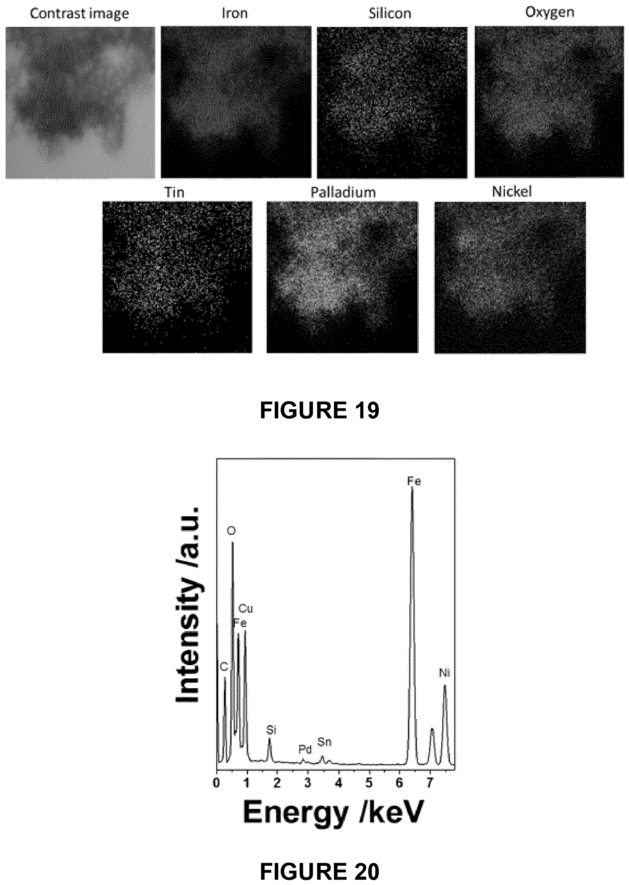

**Figure 19** shows the results of an elemental mapping analysis of the radioactive electrolessly deposited copper plated silica-coated iron oxide nanoparticles (post radioactive decay) obtained using TEM-EDX. The elemental map confirms the presence of Ni (radio-decayed product of Cu⁶⁴) in addition to Fe, O, Sn, Si and Pd signatures obtained from the underlying nanoparticles.

**Figure 20** shows the corresponding EDX spectrum of the radioactive electrolessly copper plated iron oxide nanoparticles (post radioactive decay). The EDX spectrum reveals peaks representative of oxygen (0.525 keV), iron (0.705 and 6.404 keV), silicon (1.74 keV), and tin (3.444 eV). An additional peak observed at 7.478 keV is consistent with nickel, being the radio-decayed product of ⁶⁴Cu, while the peak observed at 0.923 keV is attributed to the copper signature associated with the TEM substrate upon which the radioactive electrolessly deposited copper plated silica-coated iron oxide nanoparticles are supported during measurement.

Since the concentration of ⁶⁴Cu in the radioactive electrolessly copper plated iron oxide nanoparticles is very low (on the femtomolar level), and thus approaching the detection limit of the elemental mapping equipment, the inventors employed Radio - instant thin layer chromatography (Radio-ITLC) as a more sensitive technique to measure the ⁶⁴Cu concentration. Radio-ITLC employs a chelating agent such as ethylenediaminetetraacetic acid (EDTA), which has high affinity towards metal ions, and is capable of stripping off loosely-bound ⁶⁴Cu from the surface of the silica-coated iron oxide nanoparticle assembly, thus providing an assessment of the binding efficacy of ⁶⁴Cu to the nanoparticle.

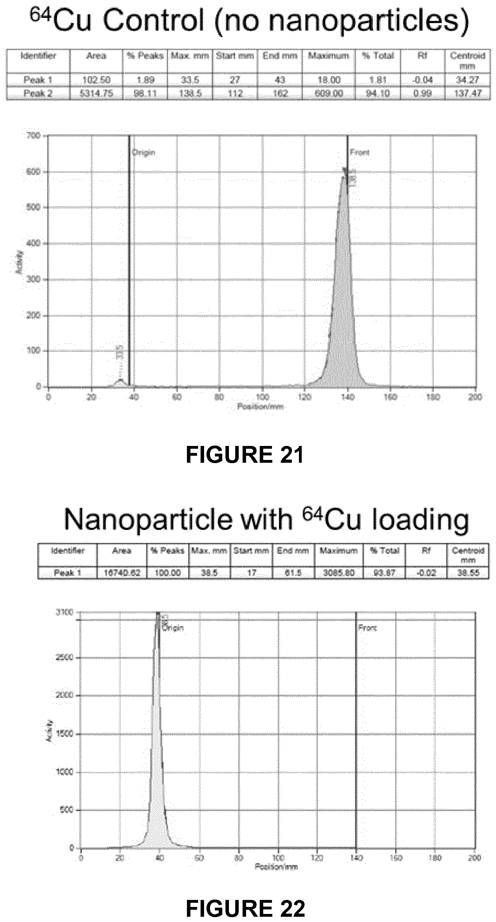

For the Radio-ITLC analysis, 2 µL of a solution of the radioactive electrolessly copper plated iron oxide nanoparticles is spotted onto one end of a piece of TLC paper. The spotted end of the TLC paper was then placed in 1 mL of 10 mM phosphate buffer containing 10% wt/vol EDTA. The poorly-bound ⁶⁴Cu and free ⁶⁴Cu ions will bind to the EDTA and migrate with the solvent front. Conversely, any ⁶⁴Cu that is rigidly bound to the nanoparticles is expected not to migrate (or show inhibited migration) due to large size of particles. The sample was allowed to migrate on TLC paper for 2 minutes or until the solvent front reached 1 cm from the top end of the TLC paper, followed by drying for 2 minutes, before being run on a Radio-ITLC reader **(****Figures 21** and **22**).

As shown in **Figure 21**, the control sample containing only free ⁶⁴Cu shows that 98.11% of the ⁶⁴Cu migrates with the solvent front.

In contrast, **Figure 22** shows that 100% of ⁶⁴Cu associated with the radioactive electrolessly copper plated iron oxide nanoparticles is bound to the silica shell, with no free ⁶⁴Cu migrating with the solvent front. Moreover, only radiation is detected at the point of origin, which indicates that a strong binding exists between the ⁶⁴Cu and the surface of the silica-coated iron oxide nanoparticles.

### Formulation

Specifically, the multimodal PET/MRI contrast agent as described above is combined with a suitable pharmaceutically acceptable excipient to provide a formulation suitable for administration to a human or non-human subject by oral, intramuscular or intravenous injection. Once formed, the formulation can then be used for imaging a region of interest in the subject using multimodal PET/MRI imaging.

In one embodiment, the pharmaceutically acceptable excipient is a buffered saline.

The multimodal PET/MRI contrast agent is present in the formulation in an amount sufficient to enhance one or more of the following images: a magnetic resonance imaging (MRI) image, a positron emission tomography (PET) image, a single photon emission computed tomography (SPECT) image and a computed tomography (CT) image.

As will be described in more detail below, the inventors have obtained good results when the multimodal PET/MRI contrast agent is present in the formulation in an amount ranging from about 0.0001% to about 25% by weight based on the total weight of the formulation.

### PET/MRI Imaging Method

To investigate the imaging capability of the as-prepared formulation, the inventors have conducted a study on a live mouse subject, the method of which will now be described.

Specifically, the formulation as described above was administered to a six week old female mouse (C57BL/6) by intravenous tail vein injection. The arrival of the multimodal PET/MRI contrast agent in the region of interest in the subject was detected by comparing the PET and/or MRI signals in the region of interest during or after administering the formulation. The PET and MRI image data of the region of interest was then collected using a multimodal PET/MRI scanner, and multimodal PET/MRI images of the region of interest were then constructed using the PET and MRI image data and visualized using suitable software. Specifically, for PET/MRI imaging of animals, the instrument used was a Bruker ClinScan PET-MR scanner comprising of a 300mm bore 7 Tesla ClinScan, running Siemens VB17, and removable PET insert containing 3 rings of 16 detector blocks with 15X15 LSO crystals (1.6 X 1.6 X 10mm) per block, at the centre of the magnet bore operating under Siemens Inveon Acquisition Workplace (IAW) software. A 72 mm ID rat body MRI rf coil inside the PET ring was used to acquire whole mouse images simultaneously with the PET acquisition.

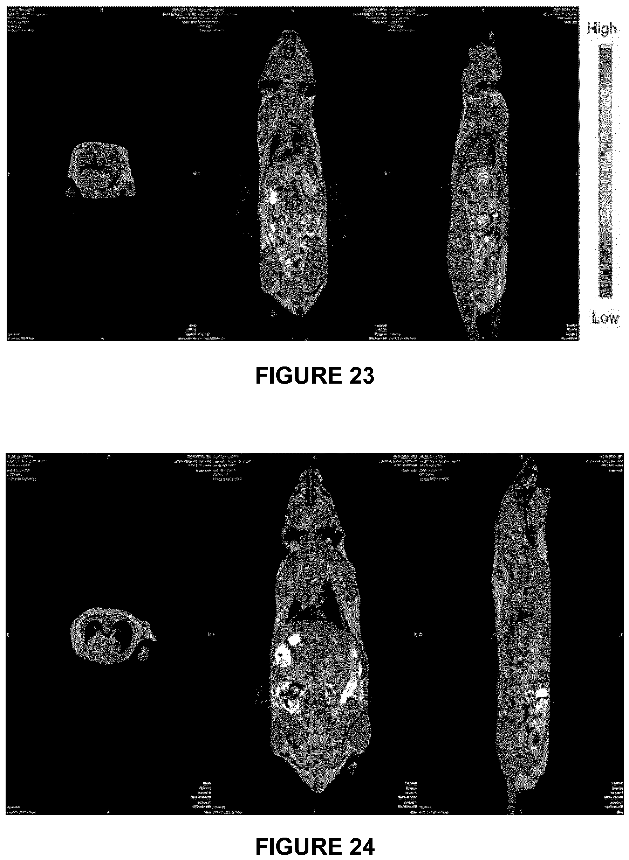

As shown in the **Figures 23** to **24**, the region of interest appears distinct from the background tissue.

**Figure 23** shows a multimodal PET/MRI image of a six week old female mouse (C57BL/6), which was exposed to the multimodal PET/MRI contrast agent [3.17 MBq equivalent of ⁶⁴Cu along with 80 µg equivalent of Fe] through tail vein injection and imaged after 18 hours.

The MRI part of the image of **Figure 23** is shown in **Figure 24**, thereby allowing a comparison between the MRI signals (the black and white regions) and the PET signals (coloured regions) observed in this study. The iron oxide component of these radioactive electrolessly deposited copper plated silica-coated iron oxide nanoparticles is a T2 contrast agent, which provides a hypo-intense (dark) signal in the MRI part (see **Figure 24**) of the image, while the ⁶⁴Cu component being a PET agent provides a 'hot'/'bright signal in the PET part of the image, represented as the coloured output.

The three images in each of these two figures show the mouse observed at different angles. The images on the left looks at the transverse view of the mouse, wherein a significant darkening is observed in the MRI signal (**Figure 24**) in the liver, overlapped with the coloured PET signal (**Figure 23**). The middle and far-right images in **Figures 23** and **24** correspond to the top-view and side-view longitudinal sections, respectively, of the mouse.

**Figures 23** and **24** clearly show that after 18 hours post-intravenous administration, most of the electrolessly deposited copper plated silica-coated iron oxide nanoparticles, as confirmed by the PET and MRI signals, are located in the liver, with a small proportion of the electrolessly deposited copper plated iron oxide nanoparticles being found in the lungs. These co-localised PET/MRI signals provide evidence that the multimodal PET/MRI contrast agent remains stable within the biological environment *in vivo,* further confirming that a strong binding exists between the superparamagnetic iron oxide nanoparticle and the ⁶⁴Cu coating, allowing for extremely good quality hybrid PET and MRI imaging capability, simultaneously.

A typical biodistribution pathway for iron-based nanoparticles intravenously administered in a live subject involves the route of travel through the heart, lungs and liver/spleen, followed subsequently by clearing or purging through the gastro-intestinal (GI) tract of the subject. However, it is often observed that coating of different materials including silica, polymers, surface functionalising agents, etc., of iron-based materials results in uncontrolled lodgement of nanomaterials, particularly in the lungs and heart, which is a major issue for *in vivo* biomedical imaging.

The observations from the multimodal PET/MRI contrast agent according to the preferred embodiment of the present invention are particularly exciting, as most of the multimodal PET/MRI contrast agent has been cleared from the lungs within 18 hours post administration. This is even more exciting as the multimodal PET/MRI contrast agent does not employ any specific strategy such as PEGylation or targeting agents to circumvent lodgement of particles in organs and tissues.

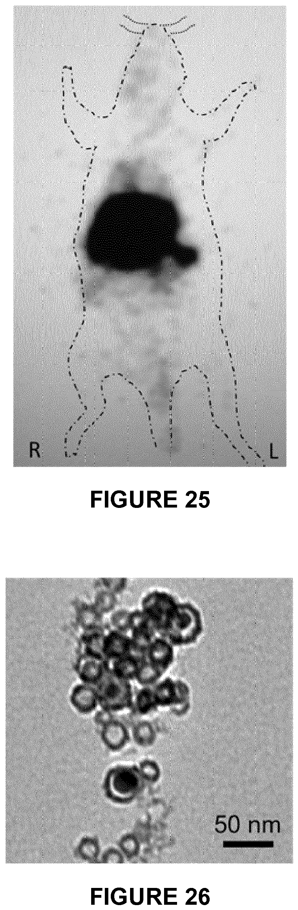

To fully understand the biodistribution profile of the multimodal PET/MRI contrast agent, the inventors intravenously administered a two day old post-synthesized multimodal PET/MRI contrast agent [2 MBq equivalent of ⁶⁴Cu along with 200 µg equivalent of Fe] into a second six week old C57BL/6 female mouse via tail vein injection, followed by PET imaging 15 minutes post-administration (see **Figure 25****).** The second mouse was immediately culled in order to harvest the various organs to determine the biodistribution of the electrolessly deposited copper plated silica-coated iron oxide nanoparticles using a radioactivity dose calibrator. At the time of death, there was a total reading of 1.66 MBq (background corrected) of radiation detected in the whole mouse.

When the second mouse was dissected, it was determined that the bulk of the observed radiation was located in the liver (1.36 MBq) and kidneys (0.11 MBq), with minor levels being detected in the heart (0.04 MBq), lungs (0.03 MBq), spleen (0.08 MBq) and blood (0.04 MBq). The observed biodistribution results support the theory that within the 15 minutes post-intravenous injection, the multimodal PET/MRI contrast agent had travelled via the bloodstream through the heart and lungs with minimal lodgement in these organs, before reaching the liver and kidneys, where the immediate process of purging the multimodal PET/MRI contrast agent from the body of the second mouse began.

Interestingly, no radiation was detected in the muscles of the second mouse. The inventors consider this to be a promising result, as it indicates that the multimodal PET/MRI contrast agent did not leach out of the gastrointestinal (GI) tract into the organs of the mouse during the procedure. Indeed, any unintended accumulation of the electrolessly deposited copper plated silica-coated iron oxide nanoparticles in the non-target organs of a live subject may result in toxicity.

A comparison of the images in **Figures 23, 24** and **25** (**Figures 23** and **24** are associated with the first mouse, while **Figure 25** is associated with the second mouse) also shows the remarkable ability of the multimodal PET/MRI contrast agent **(****Figure 23****)** in contrast to the current practice of employing only either an MRI agent (**Figure 24**) or a PET agent (**Figure 25**) towards enhancing the spatial resolution and sensitivity, which is required for efficient clinical multimodal PET/MRI imaging.

The multimodal PET/MRI contrast agent based on ⁶⁴Cu described above demonstrates that the palladium-activated silica-coated iron oxide nanoparticles may act as a template to bind a variety of potential radionuclides to create multimodal imaging and therapeutic agents.

### Indium plating of palladium-activated silica-coated iron oxide nanoparticles

To test this theory, the inventors employed the same process to electrolessly plate the palladium-activated silica-coated iron oxide nanoparticles with indium ions (In³⁺) as an example of metal ions in the +3 oxidation state. The electroless plating process in this example was carried out using a cold indium source. To minimise any variation between this cold system and the radioactive ¹¹¹In, a stock solution of acidified indium chloride (InCl₃) [pH 1.0, 10⁻⁶ M] was used as the source of metal ions. This is because ¹¹¹In is produced in indium chloride form and delivered in a hydrochloric acid solution pH 1.0 from the commercial source.

For cold indium deposition, the palladium-activated silica-coated iron oxide nanoparticles in wet pellet form are exposed to an "indium metal bath" comprising: aqueous sodium hydroxide, followed immediately by aqueous sodium potassium tartrate, subsequently followed by acidified indium chloride, immediately followed by addition of aqueous formaldehyde as the reducing agent. The reaction is then heated to 80°C for 45 minutes. Once the reaction is complete, the product is obtained, in the same manner as detailed above with respect to the non-radioactive and radioactive electrolessly deposited copper plated silica-coated iron oxide nanoparticles.

As shown in **Figure 26**, the TEM image of the resulting electrolessly indium plated silica-coated iron oxide nanoparticles shows a unique morphology, wherein rattle-like structures are obtained. Without wishing to be bound by any one particular theory, the inventors believe that the indium ions may undergo a galvanic replacement reaction with the iron oxide particle core, such that iron is replaced by indium, thereby enhancing the overall size of the nanoparticle.

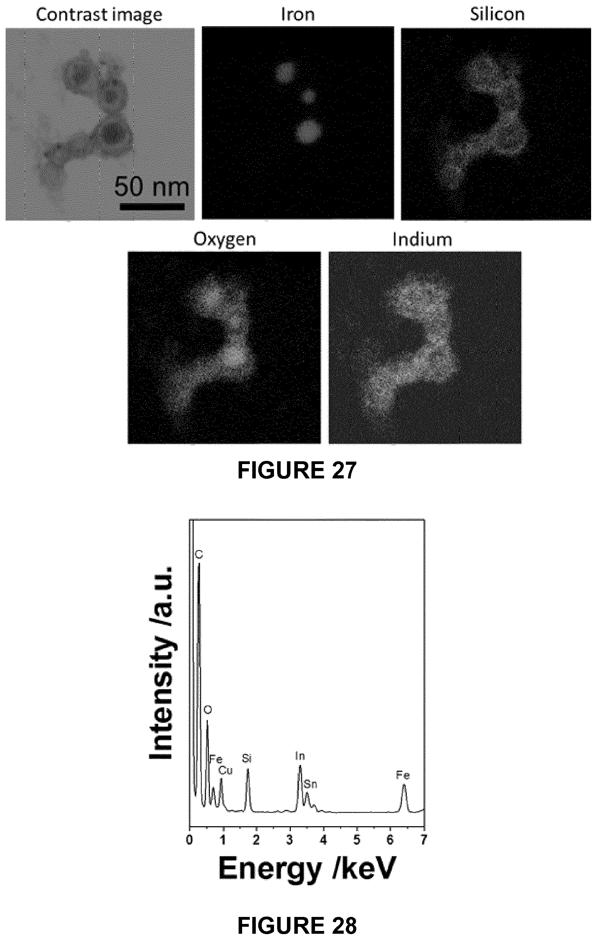

As shown in **Figure 27**, an elemental mapping analysis of these electrolessly deposited indium plated silica-coated iron oxide nanoparticles using TEM-EDX further reaffirms the presence of indium in these samples, along with the expected levels of Fe, Si, and O. The large silicon signal observed during this analysis suggests that the clearly-defined shell regions of the electrolessly deposited indium plated silica-coated iron oxide nanoparticles are most likely due to the silica shell, which assists in maintaining the integrity of these nanoparticles.

**Figure 28** shows the corresponding EDX spectrum of the electrolessly deposited indium plated silica-coated iron oxide nanoparticles. The EDX spectrum reveals peaks representative of oxygen (0.525 keV), iron (0.705 and 6.404 keV), silicon (1.74 keV), and tin (3.444 eV). An additional peak for indium is also observed at 3.286 keV confirming the successful electroless deposition of this metal. The peak observed at 0.923 keV corresponds to the copper-based TEM substrate upon which the non-radioactive electrolessly indium plated iron oxide nanoparticles are supported during measurement.

Further, while not discussed above, the inventors expect that silica shells with different thicknesses may play an influential role in controlling the amount of metal loading. Further, as shown for copper loading with respect to the non-radioactive and radioactive electrolessly deposited copper plated silica-coated iron oxide nanoparticles, indium loading may also be controlled by varying the reaction time and temperature as well as the metal salt concentration.

An ICP-MS analysis (not shown) of these electrolessly deposited indium plated silica-coated iron oxide nanoparticles, post-digestion in concentrated nitric acid reveals that the electrolessly deposited indium plated silica-coated iron oxide nanoparticles observed in **Figure 26** have the following composition: 15.8 molar equivalent of In per molar equivalent of Fe. This confirms that a large amount of indium is loaded onto the surface of the silica-coated iron oxide nanoparticles.

### Yttrium plating of palladium-activated silica-coated iron oxide nanoparticles

To further test this theory, the inventors employed the same process to electrolessly plate the palladium-activated silica-coated iron oxide nanoparticles with yttrium ions (Y³⁺) as another example of metal ions in the +3 oxidation state. The electroless plating process in this example was carried out using a cold yttrium source in the form of yttrium nitrate hexahydrate (Y(NO₃)₃•6H₂O) as the metal ion source.

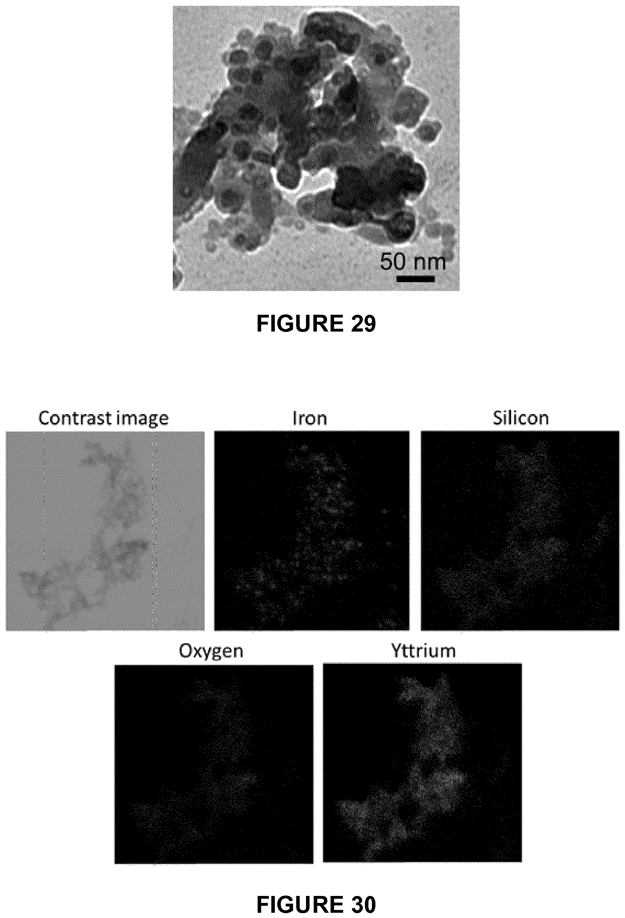

**Figure 29** shows a TEM image of the resulting electrolessly deposited yttrium plated silica-coated iron oxide nanoparticles, wherein the silica-coated iron oxide nanoparticles are seen to be encapsulated in large yttrium aggregates. That said, the inventors consider that since no effort was made to optimise the metal ion concentration needed to obtain monodispersed nanoparticles, where the metal ion concentration employed in this instance was 6 to 9 orders of magnitude lower than that employed for copper deposition as described above, those persons skilled in the relevant art may appreciate that a uniform yttrium coating may be achieved by controlling the reaction temperature, time and concentration of the yttrium source, as was seen for the other examples described above.

As shown in **Figure 30**, an elemental mapping analysis of the obtained electrolessly deposited yttrium plated silica-coated iron oxide nanoparticles using TEM-EDX confirms the presence of yttrium, along with the expected levels of Fe, Si, and O.

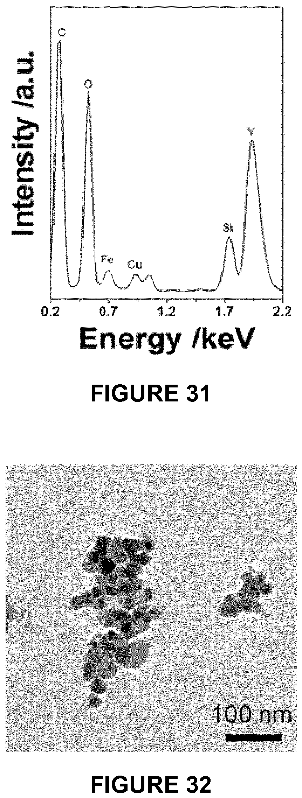

**Figure 31** shows the corresponding EDX spectrum of the electrolessly deposited yttrium plated silica-coated iron oxide nanoparticles. The EDX spectrum reveals peaks representative of oxygen (0.525 keV), iron (0.705 keV), and silicon (1.74 keV). An additional peak for yttrium is also observed at 1.922 keV confirming the successful electroless deposition of this metal. The peak observed at 0.923 keV corresponds to the copper-based TEM substrate upon which the non-radioactive electrolessly yttrium plated iron oxide nanoparticles are supported during measurement.

An ICP-MS analysis (not shown) of the electrolessly deposited yttrium plated silica-coated iron oxide nanoparticles dissolved in nitric acid, reveals the following composition: 7.7 molar equivalents of yttrium per molar equivalent of Fe, which confirms that a large amount of yttrium is loaded onto the surface of the silica-coated iron oxide nanoparticles.

### Zirconium plating of palladium-activated silica-coated iron oxide nanoparticles

To test this theory still further, the inventors employed the same process to electrolessly plate the palladium-activated silica-coated iron oxide nanoparticles with zirconium ions (Zr⁴⁺) as an example of metal ions in the +4 oxidation state. The electroless deposition process in this example was carried out using a cold zirconium source in the form of zirconium oxychloride ([Zr₄(OH)₈(H₂O)₁₆]Cl₈(H₂O)₁₂) as the metal ion source.

**Figure 32** shows a TEM image wherein the resulting electrolessly deposited zirconium plated silica-coated iron oxide nanoparticles are seen to be encapsulated in large zirconium aggregates.

Again, the inventors consider that since no effort was made to optimise the metal ion concentration needed to obtain monodispersed nanoparticles, wherein the metal ion concentration was again 6 to 9 orders of magnitude lower than that employed for copper deposition as described above, those persons skilled in the relevant art may appreciate that a uniform zirconium coating may be achieved by controlling the reaction temperature, time and concentration of the employed zirconium source, as was seen for the other examples described above.

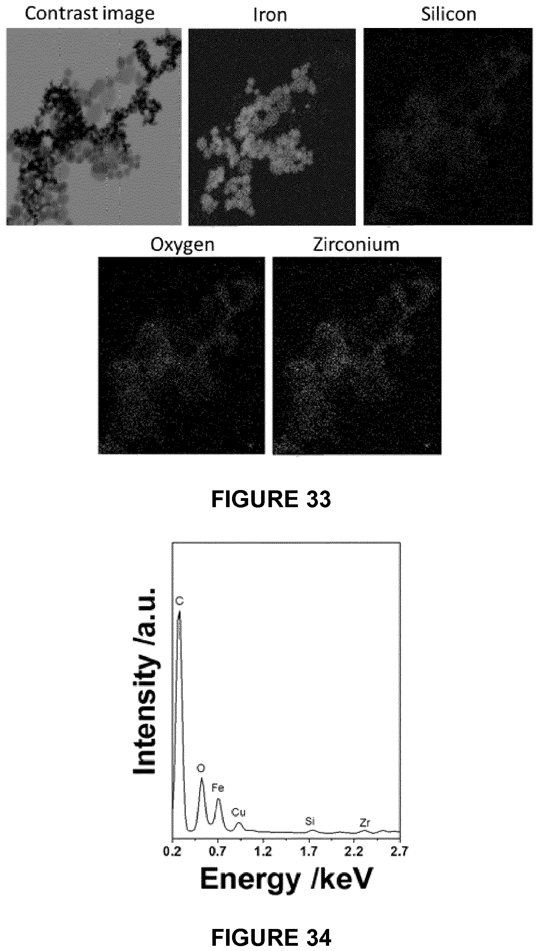

As shown in **Figure 33**, an elemental mapping analysis of the electrolessly deposited zirconium plated silica-coated iron oxide nanoparticles using TEM-EDX confirms the presence of zirconium, along with the expected levels of Fe, Si, and O.

**Figure 34** shows the corresponding EDX spectrum of the electrolessly deposited zirconium plated silica-coated iron oxide nanoparticles. The EDX spectrum reveals peaks representative of oxygen (0.525 keV), iron (0.705 keV), and silicon (1.74 keV). An additional small signature peak for zirconium is also observed at 2.304 keV, confirming the successful electroless deposition of zirconium onto the surface of the silica-coated iron oxide nanoparticles. The peak observed at 0.923 keV corresponds to the copper-based TEM substrate upon which the non-radioactive electrolessly deposited zirconium plated iron oxide nanoparticles are supported during measurement

An ICP-MS analysis (not shown) of the electrolessly deposited zirconium plated silica-coated iron oxide nanoparticles dissolved in nitric acid, reveals the following composition:0.31 molar equivalents of zirconium per molar equivalent of Fe, which confirms that zirconium can also be loaded onto the surface of the silica-coated iron oxide nanoparticles.

### Conclusion

The embodiments of the present invention described herein provide a process for the synthesis of multimodal PET/MRI contrast agents (commonly referred to as imaging agents). These multimodal PET/MRI contrast agents can either be used independently for diagnostic imaging with gamma emitters for nuclear scintigraphy, positron emission tomography (PET) imaging, magnetic resonance imaging (MRI), or independently for radionuclide therapy applications, or simultaneously for multimodal imaging applications such as PET/MRI or alternatively for simultaneous imaging and radionuclide therapy applications or theranostic applications.

The demonstrated methodology for synthesizing these multimodal PET/MRI contrast agents shows the ability to convert (reduce or hydrolyse or deposit) a number of radionuclides (or radioactive elements) from each respective oxidation group (2+, 3+ and 4+) including: ⁶⁴Cu, ⁸⁹Y, ¹¹¹In and ⁸⁹Zr, onto the surface of a carrier particle (the demonstrated carrier being oxide, more specifically being iron oxide core containing a thin silica shell) by electroless deposition or plating.

While the demonstrated systems have focussed on both PET imaging and MRI base applications as target; those persons skilled in the relevant art will be able to apply the same methodology for any radionuclide (for example, for diagnostic applications, radionuclide therapy applications and/or imaging or theranostic applications) using any carrier particle, particularly those that show a surface chemistry similar to those revealed by oxides, in general. More specifically, in the current methodology, the direct loading of radionuclides onto the surface of a silica shell within which is encapsulated a magnetic nanoparticle will also mean that those persons skilled in the relevant art will readily understand that this same proposed process can be applied to any silica/ silicate surface, irrespective of the size, shape and composition of the underlying core particle that is encapsulated by the silica shell.

The current use of radionuclides for certain applications is limited by the requirement of chelators. These complexing agents are used in order to bind radionuclides to pharmaceuticals, proteins, peptides, targeting agents or directly to biological structures such as cells. The dependence of chelators for conjugating radionuclides causes limitations such as, specific chelator to radionuclide binding, specific chelator to targeting agent binding, limited binding sites for radionuclides on targeting agent, limitation of further surface modification due to surface area of the targeting agent core structure taken up by complexing agent, resulting in a bulky final formulation, lengthy synthesis process and complex size and charge separation processing post-synthesis. In addition to this, for a cell based application the current chelator-based radionuclide formulations often suffer from a common lack of efficiency in cell retention and upon in vivo administration can lead to significant non-specific uptake. Therefore, converting a radionuclide into an inorganic particle should address each of these above mentioned limitations and in particular allow cells to retain these radionuclides. It should be appreciated that those experts in the field may like to employ the methodology presented here to further modify the invention and prepare an imaging agent with alternate capabilities (CT, ultrasound, X-ray, etc.). Further the invention may act as a carrier with or without modification. The proposed systems are also possible for tissue targeted applications, including binding of targeting agents (nucleotides, aptamers, proteins, antibodies, carbohydrate, lectins, etc.) on the nanoparticle surface using a number of methodologies available in the open literature.

The various embodiments of the invention as described herein demonstrate just one potential application of the synthesised systems (iron oxide core particle / thin silica shell / Cu⁶⁴ radionuclide and extended for ⁸⁹Zr, ⁸⁹Y, ¹¹¹In) as a multimodal PET/MRI contrast agent. This nanoparticle-based multimodal PET/MRI contrast agent system also provides scope for other applications including: multimodal PET/MRI imaging of proteins, peptides, and pharmaceuticals, together with the possibility of *in vivo* cell tracking and the like.

The described embodiments of the present invention provide a platform for further modification and use. With the design of the material in the present invention, simple substitution of the core from a T2 SPIO to a T1 agent such as gadolinium provides the possibility to further explore and exploit the virtues of the commercially available multimodal PET/MRI scanner.

### Materials and Methods

All chemicals were used as received. All chemicals and solvents were obtained from Sigma-Aldrich Australia and used without further purification.

All aqueous solutions and reagents used in the synthesis were prepared using deionized water purified using a Milli-Q^{™} reverse osmosis system unless otherwise stated.

### Preparation of iron oxide nanoparticles

Nanoparticles having a superparamagnetic iron oxide core were synthesized via a two-stage process.

In the first stage, iron oxide nanoparticles were produced by thermal decomposition according to a literature method (Park J. *et al.*, 2004)^{[2]}. Specifically, the first stage process was carried out by synthesising an iron oleate complex by dissolving 3.24 g of iron chloride and 18.25 g of sodium oleate in a solution comprised of 40 mL ethanol, 30 mL distilled water and 70 mL hexane. Once homogenized, the solution was refluxed at 70°C for 4 hours. Separation of the upper organic layer was carried out using a separatory funnel. Once separated, the iron oleate layer was washed twice with deionised water and separated again using a separatory funnel. Finally, the hexane was evaporated off leaving a waxy iron oleate complex. The iron oxide nanoparticles were formed by dissolving 10 g of the iron oleate complex in 1.77 g of oleic acid and 60.3 mL of 1-octadecene, followed by refluxing at 320°C under nitrogen for 30 minutes. The resulting mixture was then allowed to cool to room temperature.

In the second stage, the iron oxide nanoparticles obtained via the thermal decomposition method were then purified using a cleaning protocol developed by the present inventors and which forms the subject of International PCT Application No. PCT/AU2017/050981 (Gammilonghi et al.)^{[4]}*.* In this respect, the magnetic nanoparticles were purified by washing 1 mL of impure iron oxide nanoparticles with 49 mL of a first solvent composition comprising a 1:1 (vol/vol) ratio of diethyl ether and methanol, followed by magnetic separation of particles, and subsequent washing with 20 mL of a second solvent composition comprising a 1:1 (vol/vol) ratio of hexane and ethanol, before finally dispersing the purified iron oxide nanoparticles in a third solvent composition comprised of cyclohexane.

### Preparation of silica-coated iron oxide nanoparticles

Typically, 0.5 mL of polyoxyethylene (5) nonylphenyl ether (Igepal CO-520) was added to 11 mL of cyclohexane, followed by addition of 20 µL of a 2.5 mg/mL (Fe equivalent concentration) cyclohexane solution of the above-prepared and well-dispersed nanoparticles. This solution was mixed at 300 RPM of 10 minutes before 20 µL of tetraethyl orthosilicate (TEOS) was added. The solution was then allowed to mix for a further 5 minutes before100 µL of ammonia solution (28 wt. % in water) was added under stirring at 300 RPM to form a reverse microemulsion. Silica coated iron oxide nanoparticles with an overall particle size of 15 nm (± 1 nm) were obtained after 6 hours of reaction under stirring at room temperature. Once the reaction was completed, 10 mL of methanol was added to disrupt the reverse microemulsion, and the silica-coated iron oxide nanoparticles were extracted from the methanol phase using magnetic separation or centrifugation (10,000 RPM 10 minutes at room temperature).

### Preparation of tin-sensitized silica-coated iron oxide nanoparticles

The surface of the SiO₂ shell around the iron oxide nanoparticles is sensitized with tin using an acidic solution of tin chloride (SnCl₂). To achieve this, iron oxide nanoparticles containing 5 mg equivalent of iron in water are magnetically precipitated, and in the wet pellet, acidic tin chloride solution [5mL, 3 mM, pH 1.0] is added and allowed to react for 10 minutes. This results in the binding of Sn²⁺ ions to the negatively charged silica surface. After 10 minutes, the particles are rinsed twice with 5 mL of deionised water to remove any unbound Sn²⁺ ions using magnetic separation. This particle separation can also be achieved through centrifugation 10,000 RPM for 5 minutes. The resulting nanoparticles were then characterised using TEM and electron-dispersive X-ray spectroscopy (EDX).

### Preparation of palladium-activated silica-coated iron oxide nanoparticles

Seeding of palladium onto the tin-sensitised iron oxide nanoparticles is achieved by exposing the magnetically-separated particles to an aqueous palladium nitrate solution (Pd(NO₃)₂•2H₂O) [5 mL, 3mM] for 5 minutes, leading to deposition of metallic Pd⁰ nuclei on the surface of the silica shell, while simultaneously displacing the previously bound Sn²⁺ ions. During this reaction, the spontaneous formation of Pd⁰ nuclei proceeds *via* the formation of Sn⁴⁺ ions during the reduction of Pd²⁺ to Pd⁰.

### Preparation of electrolessly deposited metal plated silica-coated iron oxide nanoparticles

For metal deposition, the palladium-activated silica-coated iron oxide nanoparticles from step (**D**) are subjected to magnetic separation to isolate a wet pellet containing the palladium-activated silica-coated iron oxide nanoparticles [200 µg equivalent of Fe in the wet pellet] from the supernatant. The resulting wet pellet is exposed to a metal bath comprising: aqueous sodium hydroxide [125 µL, 1 M], followed immediately by aqueous sodium potassium tartrate [125 µL, 0.3 M], subsequently followed by an acidified metal ion source [250 µL, 10⁻⁶ M, pH 1.0], immediately followed by addition of aqueous formaldehyde [500 µL, 1M] as the reducing agent. Once the reaction is complete, the resulting particles are rinsed twice with (1 ml) deionised water using magnetic separation or centrifugation (10,000 RPM for 5 minutes) to remove any remaining reactants or by-products. The resulting pellet is then dispersed in (0.1 ml) of a suitable solvent (typically water, saline, buffer or biological growth media) through 2 minutes of sonication to obtain the electrolessly metal plated silica-coated iron oxide nanoparticles.

### Measurement of PET/MRI

Multimodal PET/MRI images were collected using a Bruker ClinScan PET-MR scanner comprising of a 300 mm bore 7 Tesla ClinScan, running Siemens VB17, and removable PET insert containing 3 rings of 16 detector blocks with 15X15 LSO crystals (1.6 X 1.6 X 10mm) per block, at the centre of the magnet bore operating under Siemens Inveon Acquisition Workplace (IAW) software. A 72 mm ID rat body MRI rf coil inside the PET ring was used to acquire whole mouse images simultaneously with the PET acquisition. (Bruker, Germany).

Multimodal PET/MRI contrast agents with a fixed radioactivity dose (3.17 MBq equivalent of ⁶⁴Cu along with 80 µg equivalent of Fe in the first mouse and 2 MBq equivalent of ⁶⁴Cu along with 200 µg equivalent of Fe in the second mouse) were injected into a six week old female mouse (C57BL/6) by intravenous tail vein injection and the intensity of the multimodal PET/MRI contrast agent both pre- and post-injection were observed at several timepoints. Multimodal PET/MRI images were visualized by Siemens Inveon Acquisition Workplace (IAW) software.

### Advantages

The multimodal PET/MRI contrast agents described in the preferred embodiments of the present invention have the potential to be used in several applications, including:
*1. Cell therapy applications*
*2*. *Diagnostic imaging*
*3*. *Therapy*
*4. Theranostic applications.*

Importantly, the radionuclide employed in the multimodal PET/MRI contrast agent of the present invention is present without the need of a chelator or linker to link the radionuclide to the nanoparticle. This offers two of the biggest advantages from the perspective of application *in vivo*: (i) a chelator-free approach allows the radionuclide [PET agent] to bind robustly to the surface of the MRI contrast agent and therefore avoid non-specific leaching during imaging, and (ii) the multimodal PET/MRI contrast agent obtained by this approach can be easily surface modified for conjugation of targeting agents such as nucleotides, aptamers, proteins, antibodies, carbohydrate, lectins, and the like.), or other labelling agents such as fluorophores, if desired.

The inventors have demonstrated that it is possible to electrolessly deposit a range of radionuclide metals from the +2, +3 and +4 oxidation states onto the surface of the as-prepared silica-coated iron oxide nanoparticles, thereby offering a generalised approach to prepare many types of imaging and therapeutic agents.

Moreover, by virtue of the synthesis being a two-step process, it is possible to prepare the silica-coated iron oxide nanoparticles beforehand, and then electrolessly deposit the radionuclide on to the surface of the silica-coated iron oxide nanoparticles at a later stage (if necessary) within standard clinical settings prior to the resulting multimodal PET/MRI contrast agent being administered to a live subject. In this respect, there is minimal loss of radioactivity.

### Other Embodiments

In other embodiments, for example, the fluorophore may be a dye impregnated within the silica (SiO₂) shell layer or a dye or indeed quantum dots coupled to the radionuclide metal layer. It will be appreciated that the binding of the fluorophore to the surface of the PET/MRI contrast agent may be achieved by any one of a number of coupling technologies.

### References

**[1]** US Patent Application No. 2004/0081617 A1 (Browitt et al.).
**[2]** Park, J., et al., Nature Materials, 2004, vol. 3, 891-895.
**[3]** US Patent Application No. 2007/0258888 A1 (Feldmann et al.).
**[4]** International PCT Patent Application No. PCT/AU2017/050981 (Gammilonghi et al.).
**[5]** Han, Y., et al., 2008, Langmuir, 24, 5842-5848.
**[6]** Anderson et al., 2016, Advanced Materials Interfaces, 2016, 3, 1500632-1-8*.*

### Definitions

Whenever a range is given in the specification, for example, a temperature range, a time range, or concentration range, all intermediate ranges and subranges, as well as all individual values included in the ranges given are intended to be included in the disclosure. It will be understood that any subranges or individual values in a range or subrange that are included in the description herein can be excluded from the claims herein.

All definitions, as defined and used herein, should be understood to control over dictionary definitions, and/or ordinary meanings of the defined terms.

Throughout this application, the term "*about*" is used to indicate that a value includes the inherent variation of error for the device, the method being employed to determine the value, or the variation that exists among the study subjects.

The indefinite articles "a" and "an," as used herein in the specification, unless clearly indicated to the contrary, should be understood to mean "at least one."

The phrase "and/or," as used herein in the specification, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined.

Spatially relative terms, such as "*internal*," "*outer*," "*beneath*," "*below*," "*lower*," "*above*," "*upper*," and the like, may be used herein for ease of description to describe one element or feature's relationship to another element(s) or feature(s) as illustrated in the Figures. Spatially relative terms may be intended to encompass different orientations of the device in use or operation in addition to the orientation depicted in the Figures.

Where the terms "comprise", "comprises", "comprised" or "comprising" are used in this specification (including the claims) they are to be interpreted as specifying the presence of the stated features, integers, steps or components, but not precluding the presence of one or more other features, integers, steps or components, or group thereof.

## Claims

1. A multimodal PET (positron emission tomography)/MRI (magnetic resonance imaging) contrast agent comprising:
a magnetic signal generating core; and
a coating portion formed at least partially over a surface of said magnetic signal generating core, wherein the coating portion comprises a plurality of layers, including an inner layer having a functionalized surface, wherein the functionalised surface is silica, and an outer layer comprising electrolessly deposited radionuclides formed on said functionalized surface.

2. A multimodal PET/MRI contrast agent according to claim 1, wherein the magnetic signal generating core is a ferromagnetic, paramagnetic or superparamagnetic signal generating core.

3. A multimodal PET/MRI contrast agent according to claim 1, wherein the magnetic signal generating core comprises:
an oxide, a mixed oxide and/or a hydroxide of at least one metal selected from the group consisting of iron (Fe), cobalt (Co), nickel (Ni), neodymium (Nd), gadolinium (Gd) and manganese (Mn);
a support material loaded with at least one metal ion selected from the group consisting of iron (Fe), cobalt (Co), nickel (Ni), neodymium (Nd), gadolinium (Gd) and manganese (Mn);
an iron-based material selected from the group consisting of iron (Fe), magnetite (Fe₃O₄), maghemite (γ-Fe₂O₃), hematite (α-Fe₂O₃), an iron alloy and a ferrite material;
an alloy of at least one metal selected from the group consisting of iron (Fe), cobalt (Co), nickel (Ni), neodymium (Nd), gadolinium (Gd) and manganese (Mn);
or any combination thereof.

4. A multimodal PET/MRI contrast agent according to claim 1, wherein the radionuclide is a positron emitter; preferably selected from the group consisting of copper (⁶⁰Cu, ⁶¹Cu, ⁶⁴Cu), scandium (⁴⁴Sc), titanium (⁴⁵Ti), iron (⁵²Fe), manganese (⁵¹Mn, ⁵²Mn), cobalt (⁵⁵Co), gallium (⁶⁶Ga, ⁶⁸Ga), arsenic (⁷²As), rubidium (^{82m} Rb), strontium (⁸³Sr), technetium (^{94m}Tc), yttrium (⁸⁶Y), zirconium (⁸⁹Zr) and indium (¹¹⁰In, ¹¹¹In); more preferably ⁶⁴Cu.

5. A multimodal PET/MRI contrast agent according to claim 1, wherein the inner layer is formed from a species selected from the group consisting of a metal, a metal oxide, a metal chalcogenide, a metal pnictogenide, a metalloid, a metalloid oxide, a metalloid chalcogenide, a metalloid pnictogenide, a self-assembled monolayer, a polyelectrolyte, a polymer, a protein, a carbohydrate and a biopolymer; preferably silica (SiO₂).

6. A multimodal PET/MRI contrast agent according to claim 1, further comprising at least one fluorophore.

7. A multimodal PET/MRI contrast agent according to claim 1, further comprising at least one quantum dot.

8. A process of synthesizing a multimodal PET (positron emission tomography)/MRI (magnetic resonance imaging) contrast agent comprising:
contacting a surface of a magnetic signal generating core with a functionalizing solution to form an inner layer at least partially over said magnetic signal generating core to define a functionalized surface;
contacting said functionalized surface with a sensitizing solution containing at least a source of bivalent tin ions to form a tin-sensitized surface;
contacting said tin-sensitized surface with an activator solution containing at least a source of palladium ions to form a palladium-activated surface; and
contacting said palladium-activated surface with an electroless plating solution containing at least a source of radionuclide ions and a reducing and/or hydrolyzing agent to convert said radionuclide ions into a layer comprising electrolessly deposited radionuclides on the palladium-activated surface.

9. A process according to claim 8, wherein the functionalizing solution comprises a tetraethyl orthosilicate (TEOS) precursor, the process further comprising:
polymerizing the TEOS precursor to form the inner layer as a silica (SiO₂) shell that at least partially encapsulates the magnetic signal generating core.

10. A process according to claim 8, wherein the sensitizing solution comprises tin (II) chloride (SnCl₂) as a source of bivalent tin ions.

11. A process according to claim 8, wherein the activator solution comprises palladium nitrate as a source of palladium ions.

12. A process according to claim 8, wherein the radionuclide is a positron emitter; preferably ⁶⁴Cu.

13. A process according to claim 8, wherein the reducing agent is formaldehyde.

14. A process according to claim 8, wherein the hydrolyzing agent is an alkali selected from the group consisting of ammonia, lithium hydroxide, sodium hydroxide, potassium hydroxide and ammonium hydroxide.

15. A process according to claim 8, wherein the electroless plating solution additionally comprises at least one additive selected from the group consisting of stabilizers, complexing agents and surfactants.

16. A method of imaging a region of interest in a patient, the method comprising:
administering to a patient a pharmaceutical formulation comprising the multimodal PET (positron emission tomography)/MRI (magnetic resonance imaging) contrast agent according to claim 1;
detecting arrival of the multimodal PET/MRI contrast agent present in the formulation in the region of interest by comparing the PET and/or MRI signals in said region of interest during or after administering said formulation;
collecting PET and MRI image data of the region of interest; and
constructing a multimodal PET/MRI image of said region of interest using the PET and MRI image data, wherein the region of interest appears distinct from the background tissue.

## Patentansprüche

1. Multimodales PET-(Positronenemissionstomographie-)/MRT-(Magnetresonanztomographie-)Kontrastmittel, das Folgendes umfasst:
einen magnetischen Signalerzeugungskern und
einen Überzugsabschnitt, der mindestens teilweise über einer Oberfläche des magnetischen Signalerzeugungskerns gebildet ist, wobei der Überzugsabschnitt eine Vielzahl von Lagen umfasst, einschließlich einer inneren Lage, die eine funktionalisierte Oberfläche aufweist, wobei die funktionalisierte Oberfläche Siliziumdioxid ist, und einer äußeren Lage, die stromlos abgeschiedene Radionuklide umfasst, gebildet auf der funktionalisierten Oberfläche.

2. Multimodales PET-/MRT-Kontrastmittel nach Anspruch 1, wobei der magnetische Signalerzeugungskern ein ferromagnetischer, paramagnetischer oder superparamagnetischer Signalerzeugungskern ist.

3. Multimodales PET-/MRT-Kontrastmittel nach Anspruch 1, wobei der magnetische Signalerzeugungskern Folgendes umfasst:
ein Oxid, ein Mischoxid und/oder Hydroxid mindestens eines Metalls, ausgewählt aus der Gruppe, die aus Eisen (Fe), Kobalt (Co), Nickel (Ni), Neodym (Nd), Gadolinium (Gd) und Mangan (Mn) besteht,
ein Trägermaterial, geladen mit mindestens einem Metallion, ausgewählt aus der Gruppe, die aus Eisen (Fe), Kobalt (Co), Nickel (Ni), Neodym (Nd), Gadolinium (Gd) und Mangan (Mn) besteht,
ein eisenbasiertes Material, ausgewählt aus der Gruppe, die aus Eisen (Fe), Magnetit (Fe₃O₄), Maghemit (γ-Fe₂O₃), Hämatit (α-Fe₂O₃), einer Eisenlegierung und einem Ferritmaterial besteht,
eine Legierung mindestens eines Metalls, ausgewählt aus der Gruppe, die aus Eisen (Fe), Kobalt (Co), Nickel (Ni), Neodym (Nd), Gadolinium (Gd) und Mangan (Mn) besteht,
oder eine beliebige Kombination derselben.

4. Multimodales PET-/MRT-Kontrastmittel nach Anspruch 1, wobei das Radionuklid ein Positronenemitter ist, vorzugsweise ausgewählt aus der Gruppe, die aus Kupfer (⁶⁰Cu, ⁶¹Cu, ⁶⁴Cu), Scandium (⁴⁴Sc), Titan (⁴⁵Ti), Eisen (⁵²Fe), Mangan (⁵¹Mn, ⁵²Mn), Kobalt (⁵⁵Co), Gallium (⁶⁶Ga, ⁶⁸Ga), Arsen (⁷²As), Rubidium (^{82m}Rb), Strontium (⁸³Sr), Technetium (^{94m}Tc), Yttrium (⁸⁶Y), Zirkonium (⁸⁹Zr) und Indium (¹¹⁰In, ¹¹¹In) besteht, insbesondere ⁶⁴Cu.

5. Multimodales PET-/MRT-Kontrastmittel nach Anspruch 1, wobei die innere Lage aus einer Spezies gebildet ist, ausgewählt aus der Gruppe, die aus einem Metall, einem Metalloxid, einem Metallchalkogenid, einem Metallpnictogenid, einem Halbmetall, einem Halbmetalloxid, einem Halbmetallchalkogenid, einem Halbmetallpnictogenid, einer selbstorganisierten Monolage, einem Polyelektrolyt, einem Polymer, einem Protein, einem Kohlenhydrat und einem Biopolymer besteht, vorzugsweise Siliziumdioxid (SiO₂).

6. Multimodales PET-/MRT-Kontrastmittel nach Anspruch 1, das ferner mindestens einen Fluorophor umfasst.

7. Multimodales PET-/MRT-Kontrastmittel nach Anspruch 1, das ferner mindestens einen Quantenpunkt umfasst.

8. Verfahren zum Synthetisieren eines multimodalen PET-(Positronenemissionstomographie-)/MRT-(Magnetresonanztomographie-)Kontrastmittels, wobei das Verfahren Folgendes umfasst:
Kontaktieren einer Oberfläche eines magnetischen Signalerzeugungskerns mit einer funktionalisierenden Lösung, um eine innere Lage mindestens teilweise über dem magnetischen Signalerzeugungskern zu bilden, um eine funktionalisierte Oberfläche zu definieren,
Kontaktieren der funktionalisierten Oberfläche mit einer sensibilisierenden Lösung, die mindestens eine Quelle von zweiwertigen Zinnionen enthält, um eine zinnsensibilisierte Oberfläche zu bilden,
Kontaktieren der zinnsensibilisierten Oberfläche mit einer Aktivatorlösung, die mindestens eine Quelle von Palladiumionen enthält, um eine palladiumaktivierte Oberfläche zu bilden, und
Kontaktieren der palladiumaktivierten Oberfläche mit einer Lösung zur stromlosen Metallabscheidung, die mindestens eine Quelle von Radionuklidionen und ein Reduktions- und/oder Hydrolysierungsmittel enthält, um die Radionuklidionen in eine Lage umzuwandeln, die stromlos abgeschiedene Radionuklide auf der palladiumaktivierten Oberfläche umfasst.

9. Verfahren nach Anspruch 8, wobei die funktionalisierende Lösung einen Tetraethylorthosilicat-(TEOS-)Vorläufer umfasst, wobei das Verfahren ferner Folgendes umfasst:
Polymerisieren des TEOS-Vorläufers, um die innere Lage als eine Siliziumdioxid-(SiO₂-)Hülle zu bilden, die den magnetischen Signalerzeugungskern mindestens teilweise einkapselt.

10. Verfahren nach Anspruch 8, wobei die sensibilisierende Lösung Zinn(II)-Chlorid (SnCl₂) als eine Quelle von zweiwertigen Zinnionen umfasst.

11. Verfahren nach Anspruch 8, wobei die Aktivatorlösung Palladiumnitrat als eine Quelle von Palladiumionen umfasst.

12. Verfahren nach Anspruch 8, wobei das Radionuklid ein Positronenemitter, vorzugsweise ⁶⁴Cu, ist.

13. Verfahren nach Anspruch 8, wobei das Reduktionsmittel Formaldehyd ist.

14. Verfahren nach Anspruch 8, wobei das Hydrolysierungsmittel ein Alkali ist, ausgewählt aus der Gruppe, die aus Ammoniak, Lithiumhydroxid, Natriumhydroxid, Kaliumhydroxid und Ammoniumhydroxid besteht.

15. Verfahren nach Anspruch 8, wobei die Lösung zur stromlosen Metallabscheidung zusätzlich mindestens einen Zuschlagstoff umfasst, ausgewählt aus der Gruppe, die aus Stabilisatoren, Komplexbildnern und grenzflächenaktiven Mitteln besteht.

16. Verfahren zur Abbildung eines Bereichs von Interesse in einem Patienten, wobei das Verfahren Folgendes umfasst:
Verabreichen einer pharmazeutischen Formulierung, die das multimodale PET-(Positronenemissionstomographie-)/MRT-(Magnetresonanztomographie-)Kontrastmittel nach Anspruch 1 umfasst, an einen Patienten,
Erfassen der Ankunft des in der Formulierung vorhandenen multimodalen PET-/MRT-Kontrastmittels in dem Bereich von Interesse durch Vergleichen der PET- und/oder MRT-Signale in dem Bereich von Interesse während oder nach dem Verabreichen der Formulierung,
Sammeln von PET- und MRT-Bilddaten des Bereichs von Interesse; und
Aufbauen eines multimodalen PET-/MRT-Bildes des Bereichs von Interesse unter Verwendung der PET- und MRT-Bilddaten, wobei der Bereich von Interesse von dem Hintergrundgewebe unterscheidbar erscheint.

## Revendications

1. Agent de contraste multimodal TEP (tomographie par émission de positrons)/IRM (imagerie par résonance magnétique), comprenant :
un noyau de génération d'un signal magnétique ;
une partie de revêtement formée au moins partiellement sur une surface dudit noyau de génération de signal magnétique, dans lequel la partie de revêtement comprend une pluralité de couches, incluant une couche interne comportant une surface fonctionnalisée, dans lequel la surface fonctionnalisée est de la silice, et une couche externe comprenant des radionucléides à dépôt autocatalytique formés sur ladite surface fonctionnalisée.

2. Agent de contraste TEP/IRM multimodal selon la revendication 1, dans lequel le noyau générateur de signal magnétique est un noyau générateur de signal ferromagnétique, paramagnétique ou superparamagnétique.

3. Agent de contraste TEP/IRM multimodal selon la revendication 1, dans lequel le noyau générateur de signal magnétique comprend :
un oxyde, un oxyde mixte et/ou un hydroxyde d'au moins un métal choisi dans le groupe constitué de fer (Fe), de cobalt (Co), de nickel (Ni), de néodyme (Nd), de gadolinium (Gd) et de manganèse (Mn) ;
un matériau de support chargé d'au moins un ion métallique sélectionné dans le groupe constitué de fer (Fe), de cobalt (Co), de nickel (Ni), de néodyme (Nd), de gadolinium (Gd) et de manganèse (Mn) ;
un matériau à base de fer sélectionné dans le groupe constitué de fer (Fe), de magnétite (Fe₃O₄), de maghémite (y-Fe₂O₃), d'hématite (α-Fe₂O₃), d'un alliage de fer et d'un matériau de ferrite ;
un alliage d'au moins un métal sélectionné dans le groupe constitué de fer (Fe), de cobalt (Co), de nickel (Ni), de néodyme (Nd), de gadolinium (Gd) et de manganèse (Mn) ;
ou une quelconque combinaison de ceux-ci.

4. Agent de contraste TEP/IRM multimodal selon la revendication 1, dans lequel le radionucléide est un émetteur de positrons ; sélectionné de préférence dans le groupe constitué de cuivre (⁶⁰Cu, ⁶¹Cu, ⁶⁴Cu), de scandium (⁴⁴Sc), de titane (⁴⁵Ti), de fer (⁵²Fe), de manganèse (⁵¹Mn, ⁵²Mn), de cobalt (⁵⁵Co), de gallium (⁶⁶Ga, ⁶⁸ Ga), d'arsenic (⁷²As), de rubidium (^{82m}Rb), de strontium (⁸³Sr), de technétium (^{94m}Tc), d'yttrium (⁸⁶Y), de zirconium (⁸⁹Zr) et d'indium (¹¹⁰In, ¹¹¹In) ; de préférence ⁶⁴Cu.

5. Agent de contraste TEP/IRM multimodal selon la revendication 1, dans lequel la couche interne est formée à partir d'une espèce sélectionnée dans le groupe constitué d'un métal, d'un oxyde métallique, d'un chalcogénure métallique, d'un pnictogénure métallique, d'un métalloïde, d'un oxyde de métalloïde, d'un chalcogénure de métalloïde, d'un pnictogénure de métalloïde, d'une monocouche auto-assemblée, d'un polyélectrolyte, d'un polymère, d'une protéine, d'un glucide et d'un biopolymère, de préférence la silice (SiO₂).

6. Agent de contraste TEP/IRM multimodal selon la revendication 1, comprenant en outre au moins un fluorophore.

7. Agent de contraste TEP/IRM multimodal selon la revendication 1, comprenant en outre au moins un point quantique.

8. Procédé de synthèse d'un agent de contraste multimodal TEP (tomographie par émission de positrons)/IRM (imagerie par résonance magnétique), comprenant les étapes suivantes :
mise en contact d'une surface d'un noyau générateur de signal magnétique avec une solution de fonctionnalisation pour former une couche interne au moins partiellement sur ledit noyau générateur de signal magnétique pour définir une surface fonctionnalisée ;
mise en contact de ladite surface fonctionnalisée avec une solution sensibilisante contenant au moins une source d'ions d'étain bivalents pour former une surface sensibilisée à l'étain ;
mise en contact de ladite surface sensibilisée à l'étain avec une solution d'activateur contenant au moins une source d'ions palladium pour former une surface activée au palladium ; et
mise en contact de ladite surface activée au palladium avec une solution de placage autocatalytique contenant au moins une source d'ions radionucléides et un agent réducteur et/ou hydrolysant pour convertir lesdits ions radionucléides en une couche comprenant des radionucléides déposés par placage autocatalytique sur la surface activée au palladium.

9. Procédé selon la revendication 8, dans lequel la solution de fonctionnalisation comprend un précurseur d'orthosilicate de tétraéthyle (TEOS), le procédé comprenant en outre l'étape suivante :
polymérisation du précurseur de TEOS pour former la couche interne sous la forme d'une enveloppe de silice (SiO₂) qui encapsule au moins partiellement le noyau générateur de signal magnétique.

10. Procédé selon la revendication 8, dans lequel la solution de sensibilisation comprend du chlorure d'étain (II) (SnCl₂) comme source d'ions étain bivalents.

11. Procédé selon la revendication 8 dans lequel la solution d'activateur comprend du nitrate de palladium comme source d'ions palladium.

12. Procédé selon la revendication 8, dans lequel le radionucléide est un émetteur de positrons ; de préférence ⁶⁴Cu.

13. Procédé selon la revendication 8, dans lequel l'agent réducteur est le formaldéhyde.

14. Procédé selon la revendication 8, dans lequel l'agent hydrolysant est un alcali sélectionné dans le groupe constitué de l'ammoniac, l'hydroxyde de lithium, l'hydroxyde de sodium, l'hydroxyde de potassium et l'hydroxyde d'ammonium.

15. Procédé selon la revendication 8, dans lequel la solution de placage autocatalytique comprend en outre au moins un additif sélectionné dans le groupe constitué de stabilisants, d'agents complexants et de tensioactifs.

16. Procédé de réalisation d'une image d'une région d'intérêt chez un patient, le procédé comprenant les étapes suivantes :
administration à un patient d'une formulation pharmaceutique comprenant l'agent de contraste multimodal TEP (tomographie par émission de positrons)/IRM (imagerie par résonance magnétique) selon la revendication 1 ;
détection de l'arrivée de l'agent de contraste multimodal TEP/IRM présent dans la formulation dans la région d'intérêt en comparant les signaux TEP et/ou IRM dans ladite région d'intérêt pendant ou après l'administration de ladite formulation ;
collecte de données d'image TEP et IRM de la région d'intérêt ; et
construction d'une image TEP/IRM multimodale de ladite région d'intérêt en utilisant les données d'image TEP et IRM, dans lequel la région d'intérêt apparait distincte du tissu de fond.
